(19) **Europäisches Patentamt / European Patent Office / Office européen des brevets**

(11) **EP 2 158 326 B1**

(12) **EUROPEAN PATENT SPECIFICATION**

(45) Date of publication and mention
of the grant of the patent:
**20.06.2012 Bulletin 2012/25**

(51) Int Cl.:
**C12Q 1/00** (2006.01)    **G01N 33/68** (2006.01)

(21) Application number: **08768697.8**

(22) Date of filing: **20.06.2008**

(86) International application number:
**PCT/US2008/007772**

(87) International publication number:
**WO 2008/156867 (24.12.2008 Gazette 2008/52)**

(54) **BIOMARKERS FOR RHEUMATOID ARTHRITIS**

BIOMARKER FÜR RHEUMATOIDE ARTHRITIS

BIOMARQUEURS DE LA POLYARTHRITE RHUMATOÏDE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **21.06.2007 US 945526 P**

(43) Date of publication of application:
**03.03.2010 Bulletin 2010/09**

(73) Proprietors:
• **The Board of Trustees of the Leland Stanford Junior University Palo Alto CA 94306-1106 (US)**
• **The Government of the United States of America, as represented by The Department of Veterans Affairs Washington, DC 20420 (US)**
• **The Regents of the University of Colorado Denver, CO 80203 (US)**

(72) Inventors:
• **ROBINSON, William H. Palo Alto, California 94304-1207 (US)**
• **HOLERS, Michael Aurora, Colorado 80045 (US)**
• **DEANE, Kevin Aurora, Colorado 80045 (US)**

(74) Representative: **Brasnett, Adrian Hugh Mewburn Ellis LLP 33 Gutter Lane London EC2V 8AS (GB)**

(56) References cited:
WO-A1-2006/008183    WO-A2-2005/064307
US-A1- 2006 188 913    US-A1- 2007 020 691

• DEANE KEVIN D ET AL: "Specific cytokine and chemokine increases precede the appearance of anti-cyclic citrullinated protein (Anti-CCP) antibodies and rheumatoid factor (RF) in the pre-clinical period of rheumatoid arthritis (RA) development." ARTHRITIS & RHEUMATISM, vol. 54, no. 9, Suppl. S, September 2006 (2006-09), page S508, XP008121643 & 70TH ANNUAL SCIENTIFIC MEETING OF THE AMERICAN-COLLEGE-OF-RHEUMATOLOG Y/41ST ANNUAL SCIENTIFIC MEETIN; WASHINGTON, DC, USA; NOVEMBER 10 -15, 2006 ISSN: 0004-3591
• NIELEN M M J ET AL: "Simultaneous development of acute phase response and autoantibodies in preclinical rheumatoid arthritis" ANNALS OF THE RHEUMATIC DISEASES, vol. 65, no. 4, April 2006 (2006-04), pages 535-537, XP008121647 ISSN: 0003-4967
• HUEBER WOLFGANG ET AL: "Proteomic analysis of secreted proteins in early rheumatoid arthritis: anti-citrulline autoreactivity is associated with up regulation of proinflammatory cytokines" ANNALS OF THE RHEUMATIC DISEASES, vol. 66, no. 6, 10 August 2006 (2006-08-10), pages 712-719, XP002579785 ISSN: 0003-4967
• HITCHON CAROL A ET AL: "A distinct multicytokine profile is associated with anti-cyclical citrullinated peptide antibodies in patients with early untreated inflammatory arthritis" JOURNAL OF RHEUMATOLOGY, vol. 31, no. 12, December 2004 (2004-12), pages 2336-2346, XP008121646 ISSN: 0315-162X

**(Cont. next page)**

EP 2 158 326 B1

- HUEBER WOLFGANG ET AL: "Antigen microarray profiling of autoantibodies in rheumatoid arthritis" ARTHRITIS & RHEUMATISM, JOHN WILEY & SONS, INC, US LNKD- DOI:10.1002/ART.21269, vol. 52, no. 9, 1 September 2005 (2005-09-01), pages 2645-2655, XP009101906 ISSN: 0004-3591
- CRONSTEIN BRUCE N: "Interleukin-6--a key mediator of systemic and local symptoms in rheumatoid arthritis." BULLETIN OF THE NYU HOSPITAL FOR JOINT DISEASES 2007 LNKD-PUBMED:17708739, vol. 65 Suppl 1, 2007, pages S11-S15, XP002579786 ISSN: 1936-9719
- DATABASE EMBASE [Online] ELSEVIER SCIENCE PUBLISHERS, AMSTERDAM, NL; 2005, CULIC S: "Cytokines and autoimmune disseases" XP002579787 Database accession no. EMB-2008183396 & PAEDIATRIA CROATICA, SUPPLEMENT 2005 HR, vol. 49, no. 1, 2005, pages 148-161, ISSN: 1330-724X

**Description**

BACKGROUND OF THE INVENTION

Autoimmune disease occurs when a specific adaptive immune response is mounted against self antigens. The consequence is that the effector pathways of immunity cause chronic inflammatory injury to tissues, which may prove lethal. Autoimmunity may be initiated by the activation of antigen-specific T cells, although the specific triggering mechanism remains unknown. Specific genes found within the major histocompatability complex (MHC) as well as at other immunoregulatory loci play key roles in determining the susceptibility of individuals to develop autoimmune diseases, likely because of their ability to modulate adaptive T and B cell immune responses. T-cell responses to self antigens can inflict tissue damage through cytotoxic T-cell responses, inappropriate activation of non-specific effector cells, and inappropriate T-cell help to B cells. Diseases in which these actions of T cells are likely to be important include type I IDDM, rheumatoid arthritis, and multiple sclerosis. Affected tissues in patients with these diseases are heavily infiltrated with T lymphocytes and activated macrophages.

A variety of inflammatory autoimmune diseases may be mediated by $T_H1$ cells responding to self antigens, and by cytokines involved in such an immune response. EAE, for example, may be caused by $T_H1$ cells specific for myelin proteins. Rheumatoid arthritis may be caused by $T_H1$ cells specific for antigens present in joints. Engagement with this antigen triggers the T cells to release lymphokines that initiate local inflammation within the joint. This causes swelling, accumulation of polymorphonuclear leukocytes and macrophages, and damage to cartilage, leading to the destruction of the joint. Rheumatoid arthritis is a complex disease and also involves antibodies, often including an IgM anti-IgG autoantibody called rheumatoid factor.

Although the etiology of most autoimmune diseases is unknown, it has become apparent that interaction and communication between regulatory and effector cells of the immune system is important in triggering and maintaining autoimmune inflammation, as well as potentially causing tissue damage tissue damage. As key elements of this communication network, cytokines and chemokines orchestrate the recruitment, survival, expansion, effector function and contraction of autoreactive lymphocytes in autoimmunity.

Cytokines are messenger molecules produced by B cells, T cells, macrophages, dendritic cells and other immune and host cells. Cytokines play roles in the pathogenesis of rheumatoid arthritis, multiple sclerosis and other autoimmune diseases. Cytokines include chemokines, interleukins, lymphokines, growth factors, angiogenesis factors, and other secreted and cell surface molecules that transmit signals to other cells. Cytokines include, but are not limited to, TNFα, INFγ, IL-1, IL-2, IL-4 IL-6, IL-8/CXCL8 IL-10, IL-12, IL-13, IL-15, IL-17, IL-18, IL-23, RANTES/CCL5, IP-10/CXCL10, eotaxin/CCL11, MCP-1/CCL2, MIP-1α/CCL4, growth factors such as GM-CSF, VEGF, PDGF, IGF; other secreted molecules include proteases such as metalloproteinases (MMPs), and their tissue inhibitors (TIMPs). Blockade of several of these with biological agents (monoclonal antibodies and soluble receptors), including TNFα (with etanercept, infliximab and adalimumab), IL-1 (with Anakinra) and IL-6 (Tocilizumab, currently in trials), have already provided therapeutic benefit in autoimmune diseases.

The concept of multiparameter testing for prediction analysis is generalizable to many clinical applications where actionable a priori assessments of treatment responses are sought. Analysis of multiple parameters is often necessary to meet clinical requirements for a reliable biomarker that provides for prediction of clinical disease onset and disease severity. Such analysis may allow the assessment of an individual's need for preventive therapy as well as his/her chances to respond favorably to treatment with a specific drug, and facilitate the design of a personalized therapeutic plan.

The development and analysis of such biomarkers is of great clinical interest. Such biomarkers could enable the physician to identify patients at risk of developing autoimmune disease, and thus institute early intervention. There is tremendous interest in early intervention, including multiple clinical trials to investigate therapeutic agents in such regimens.

Publications

Autoantibody profiles and uses thereof are described in U.S. Patent application, publication US-2003-0003516-A1.

Deane et al, Arthritis & Rheumatism, vol. 54, no. 9, Suppl. S, September 2006 (2006-09), page 8508" describe specific cytokine and chemokine increases precede the appearance of anti-cyclic citrullinated protein (Anti-CCP) antibodies and rheumatoid factor (RF) in the preclinical period of rheumatoid arthritis (RA) development.

Nielen et al, Ann Rheum Dis. 2006 Apr;65(4):535-537, describe the simultaneous development of acute phase response and autoantibodies in preclinical rheumatoid arthritis.

SUMMARY OF THE INVENTION

The present invention relates to compositions and methods for prognostic classification of individuals into subtypes with respect to development of overt rheumatoid arthritis (RA), which is an autoimmune disease, which subtypes are informative of the patient's probability of developing overt RA. The patterns of circulating levels of serum autoantibodies and cytokines identified herein provides for a signature pattern that can discriminate individuals who have a high probability of developing overt RA from those who have a low probability of developing overt RA. Assessment of this signature pattern of autoantibodies and cytokines in a patient thus allows improved care by indicating where therapeutic action may be required, at an early stage of disease.

The present invention thus provides a method for the classification of an individual into subtypes, which subtypes are informative of the individual's probability of developing overt rheumatoid arthritis (RA), the method comprising:

determining a cytokine signature pattern for increased levels of IL-6; and an autoantibody signature pattern for increased levels of at least 3 autoantibodies from a sample obtained from said autoimmune disease patient, wherein said autoantibodies bind to an antigen selected from citrullinated epitopes derived from histone 2A (H2A), fibrinogen (Fibrgn and hFib), fillagrin (cfc-1 and cf48-65), vimentin (Vim65-77cit and Vim1-20cit), and ApoE(277-296)cit; comparing said cytokine and autoantibody signature pattern with a control signature pattern; wherein a statistically significant match with a positive pattern for a pre-disease subtype or a statistically significant difference from a normal pattern for said disease is indicative that said patient is at risk for developing overt rheumatoid arthritis.

Further embodiments of the methods are set out in the appended claims

Circulating cytokines, including IL-6, are identified and described herein that are differentially expressed in patients at a very early stage of autoimmune disease that is prior to development of overt autoimmune disease. Such cytokines are analyzed in conjunction with detection of autoantibody markers as defined in claim 1, and optionally with further markers including but not limited to rheumatoid factor (RF) and anti-cyclic citrullinated peptide (CCP). Circulating cytokine markers identified herein include IP-10 (CXCL-10), MCP-1, CRP, eotaxin, GM-CSF, FGF-2, Flt-3 ligand, IL-10, IL-12 p40, IL-12 p70, IL-15, IL-1$\alpha$, IL-1$\beta$, IL-6, and TNF$\alpha$. In some embodiments of the invention, IP-10 and MCP-1 are used for the prognostic classification of individuals at early stages of disease, e.g. in individuals that are anti-CCP negative and at risk of developing rheumatoid arthritis.

The method of the invention can classify patients as belonging to specific disease classes. For example, with regard to pre-disease states, one can classify individuals as: normal (N); RF+ anti-CCP- and pre-disease (RFPD); RF+ anti-CCP- not pre-disease (RFNPD); anti-CCP+ RF- pre-disease (CCPRFNPD), anti-CCP+ RF- not pre-disease (CCPPRFNNPD), RF+ anti-CCP+ pre-disease (RFCCPPD), and overt RA (RA). Overt RA can be further classified into very early disease (< 6 months of symptoms), early disease (< 2 years of symptoms), and late disease (> 2 years of symptom). Classifications can be modified by the expanded autoantibody profiles as determined by array analysis (both genetic and proteomic), the presence or absence of cytokines, chemokines, non-specific markers of inflammation (CRP, ESR), or genetic risk (ie, presence or absence of certain HLA alleles or susceptibility alleles from genes such as PTPN22 and CTLA4). Certain characteristics of environmental exposures or answers to standard questionnaires regarding the presence or absence of clinical symptoms may modify assessment of risk of overt disease or pre-disease states. Overt disease characterization can include levels of disease activity, degree of joint destruction and disability, and presence or absence of extra-articular features.

In one embodiment of the invention, the expression profile of a panel of proteins is evaluated for conditions indicative of various early and pre-disease stages of autoimmunity and clinical sequelae thereof. Such a panel provides a level of discrimination not found with individual markers. In one embodiment, the expression profile is determined by measurements of protein concentrations or amounts.

Methods of analysis may include, without limitation, utilizing a dataset to generate a predictive model, and inputting test sample data into such a model in order to classify the sample according to an autoimmune classification, where the classification is selected from the group consisting of an autoimmune disease classification, a healthy classification, a pre-disease classification, and classifying the sample according to the output of the process. In some embodiments, such a predictive model is used in classifying a sample obtained from a mammalian subject by obtaining a dataset associated with a sample, wherein the dataset comprises at least three, or at least four, or at least five protein markers selected from the group comprising or consisting of IP-10 (CXCL-10), MCP-1, CRP, eotaxin, GM-CSF, FGF-2, Flt-3 ligand, IL-10, IL-12 p40, IL-12 p70, IL-15, IL-1$\alpha$, IL-1$\beta$, IL-6, and TNF$\alpha$. The data optionally includes a profile for other indices including: genetic markers (including HLA alleles, PTPN22, CTLA4 risk alleles, etc), clinical manifestation of autoimmune disease (joint involvement, degree of disability, etc), expression of autoantibodies (including RF and anti-CCP, etc), and non-specific measures of inflammation (including ESR, CRP, etc).

A predictive model of the invention utilizes quantitative data from one or more sets of marker as set out in the claims. In some embodiments a predictive model provides for a level of accuracy in classification; i.e. the model satisfies a desired quality threshold. A quality threshold of interest may provide for an accuracy or AUC of a given threshold, and either or both of these terms (AUC; accuracy) may be referred to herein as a quality metric. A predictive model may provide a quality metric, e.g. accuracy of classification or AUC, with varying cut-offs of biomarker levels selected to provide a desired balance of sensitivity and selectivity.

BRIEF DESCRIPTION OF THE DRAWINGS

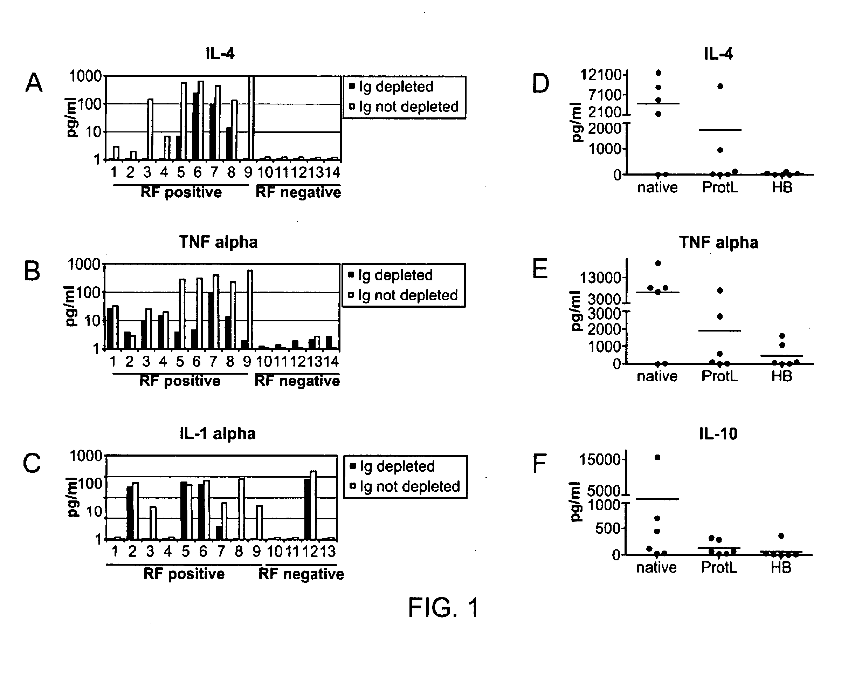

Figure 1. Impact of immunoglobulin depletion and blocking of heterophilic antibodies on quantification of cytokines: optimization of methods for the bead-array. A-C. Serum samples from 14 patients with established RA (9 RF seropositive, 5 RF seronegative) were either depleted of immunoglobulins by immunoprecipitation using protein L-sepharose beads, or used untreated, followed by analysis on the multiplex cytokine assay. Representative results are shown for IL-4 (A), TNFα (B), and IL-10 (C). Concentrations in pg/ml are shown on a logarithmic scale on the left, RF seropositive and RF seronegative samples are labeled on the bottom of each panel, left columns represent measurements in immunoglobulin-depleted serum, right columns represent measurements in undepleted serum. Immunodepletion was performed by incubation of 100 μl of serum with 25 μl of protein L-sepharose beads (Pierce Biotechnologies, Rockford, IL) for 30 min at 4° C, followed by 30 sec centrifugation at 14k RPM and removal of the supernatant for cytokine analysis. D-F. Serum samples from 4 ARAMIS patients with RF seropositive and 2 patients with RF seronegative RA, were analyzed by multiplex cytokine assay. Serum samples were either untreated (native), or pretreated by (i) incubation with protein L-sepharose beads to remove immunoglobulin; and (ii) a blocking agent (HeteroBlock™) at 1:175 dilution, followed by sample analysis on the multiplex cytokine assay. Each dot represents an individual sample. Concentrations in ng/ml are indicated on a linear scale on the left of each graph. Different sample treatment groups are labeled below the respective columns. RF, rheumatoid factor; Ig, immunoglobulin; ProtL, protein L-sepharose beads; HB, Heteroblock™

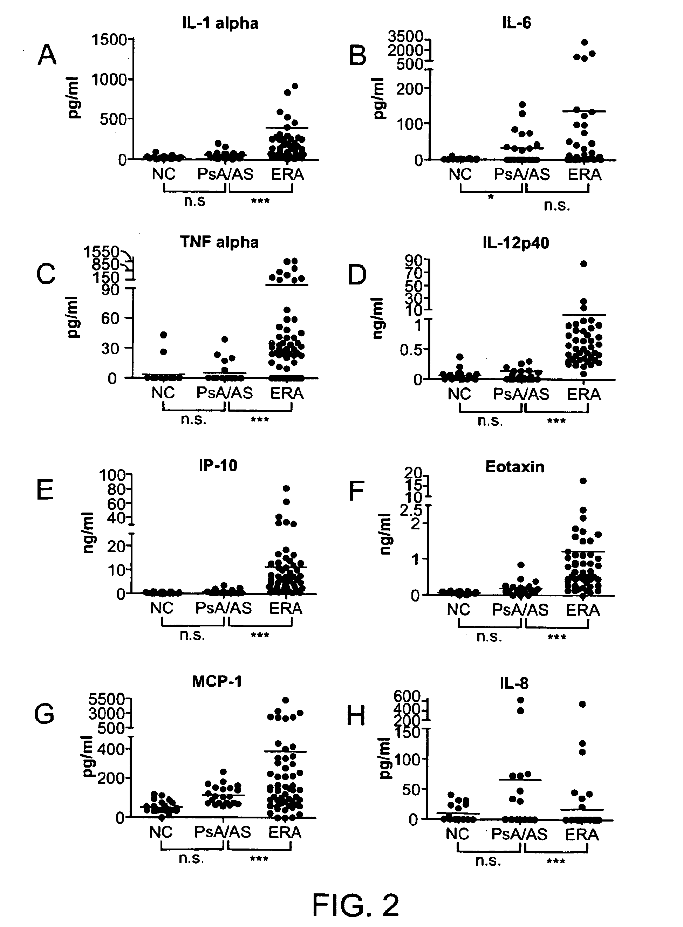

Figure 2. Comparison of cytokine concentrations in healthy individuals, patients with PsA and AS, and early RA by multiplex bead-array. Serum samples from patients with early RA (n = 56), PsA and AS (n = 21) and from healthy subjects (n = 19) were analyzed by multiplex cytokine assay using Heteroblock. Representative results are shown for IL-1α (A), IL-6 (B), TNFα (C), IL-12p40 (D), IP-10/CXCL10 (E), Eotaxin/CCL11 (F), MCP-1/CCL2 (G), and IL-8/CXCL8 (H). Horizontal bars represent medians with percentiles for each column.

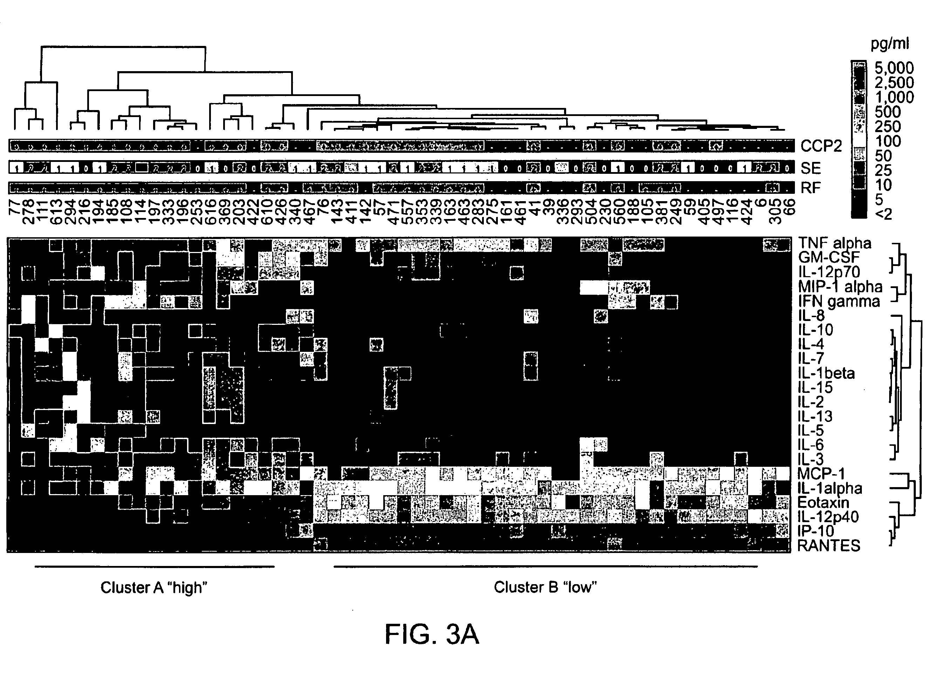

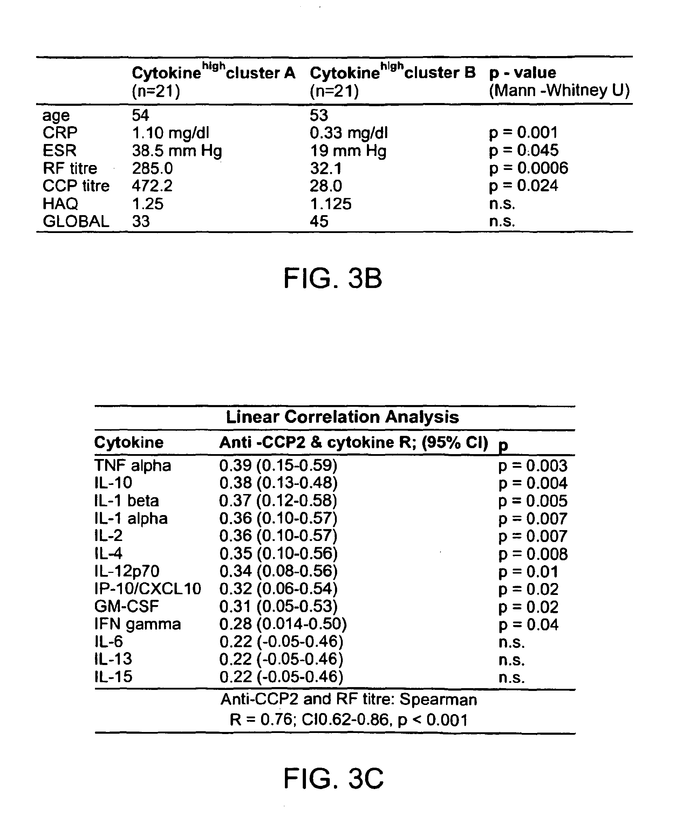

Figure 3. Blood cytokine profiles stratify early RA patients. We applied a bead-based array using an optimized protocol to profile cytokines in RA serum samples. (A) Array results are displayed as a heat map after hierarchical clustering of all data points to visualize the spectrum of cytokine levels for each patient. Columns represent individual patients, labeled on the top. Red represents the highest cytokine values. For each patient, the number of copies of the SE, RF status, and CCP2 ELISA reactivity are indicated across the top of the panel. Rows representing individual cytokine levels are labeled on the right side of the panel. (B) Comparison between patients and their disease activity parameters in "Clusters A high" and "Cluster B low". (C) Linear correlation analysis was performed to determine correlations between anti-CCP2 and cytokine concentrations. Correlation coefficients for selected individual pairs are displayed in descending order.

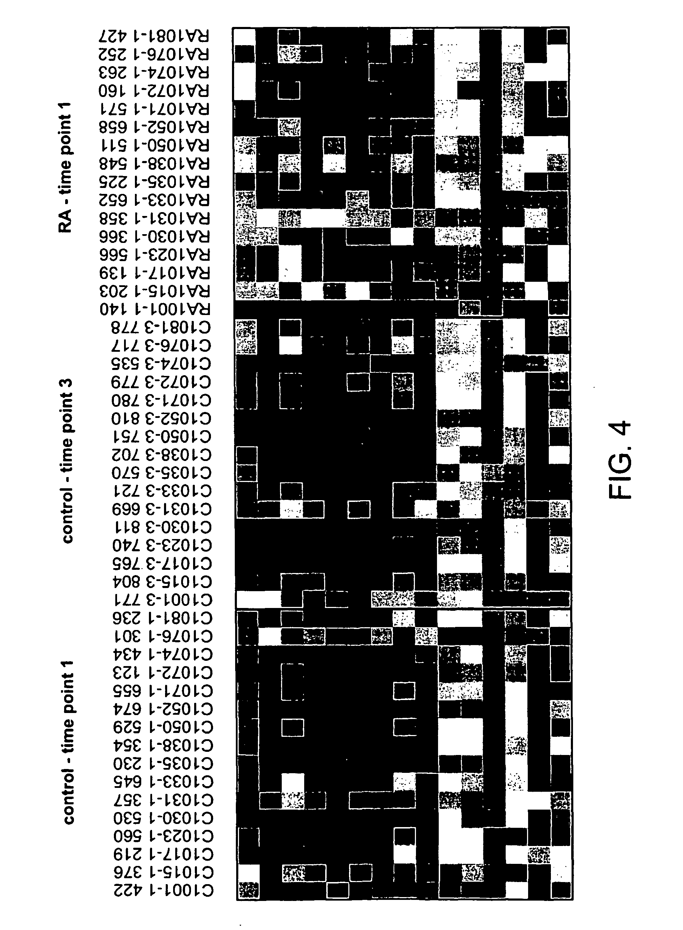

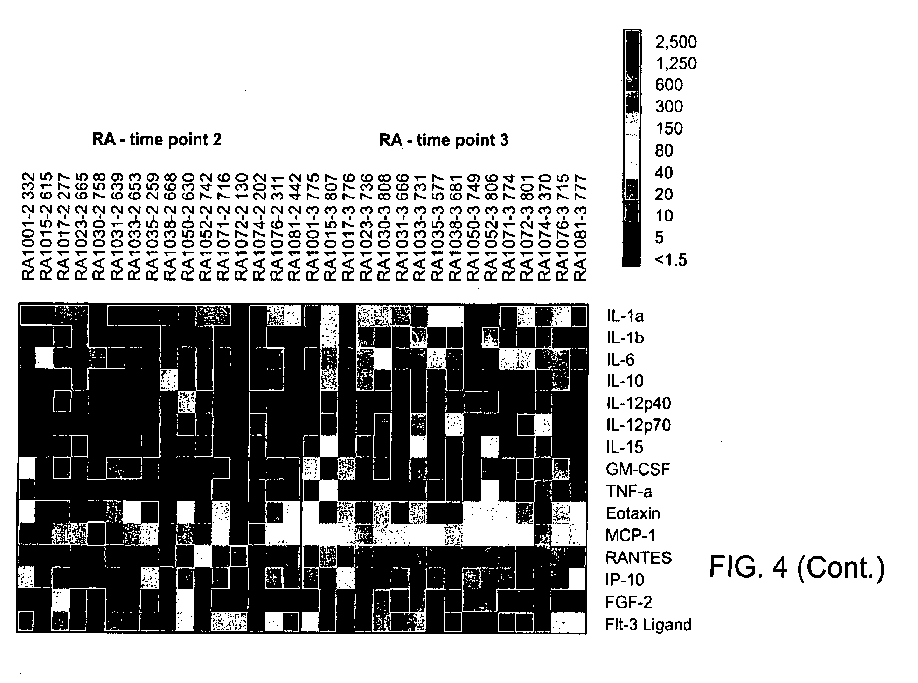

Figure 4. Increased cytokines are present in pre-disease samples derived from patients that developed RA. Cytokine and chemokine profiling was performed using a bead-array and the methods described in Figure 1. Serial pre-disease sera were characterized from 16 patients that subsequently developed RA. Elevations in levels of eotaxin (CCL11), IL-1alpha, GM-CSF, and MCP-1 (CCL2) were found in pre-clinical samples compared to controls. TP1, TP2 and TP3 represent time points 1, 2 and 3, respectively, with TP1 representing the earliest samples obtained from the patients that subsequently developed RA.

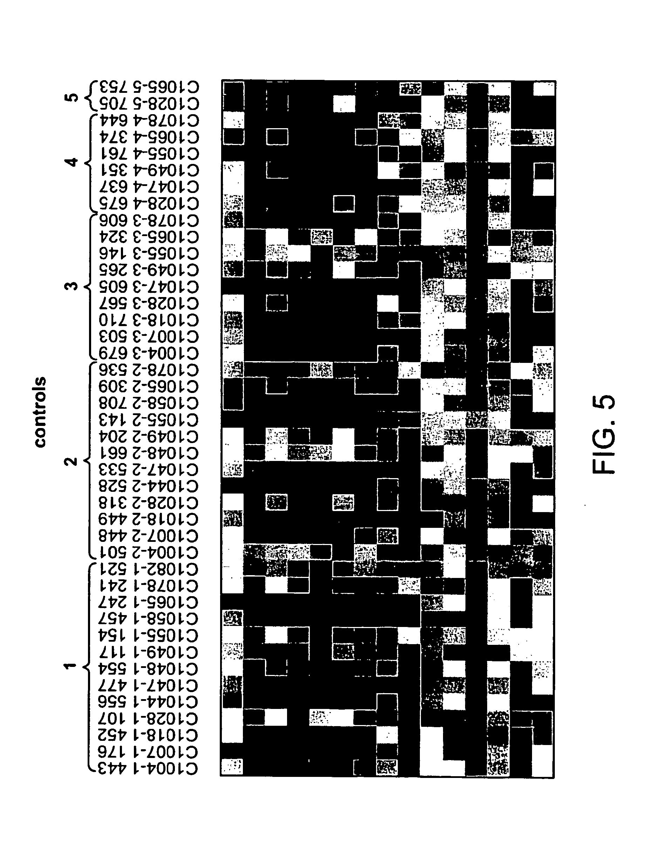

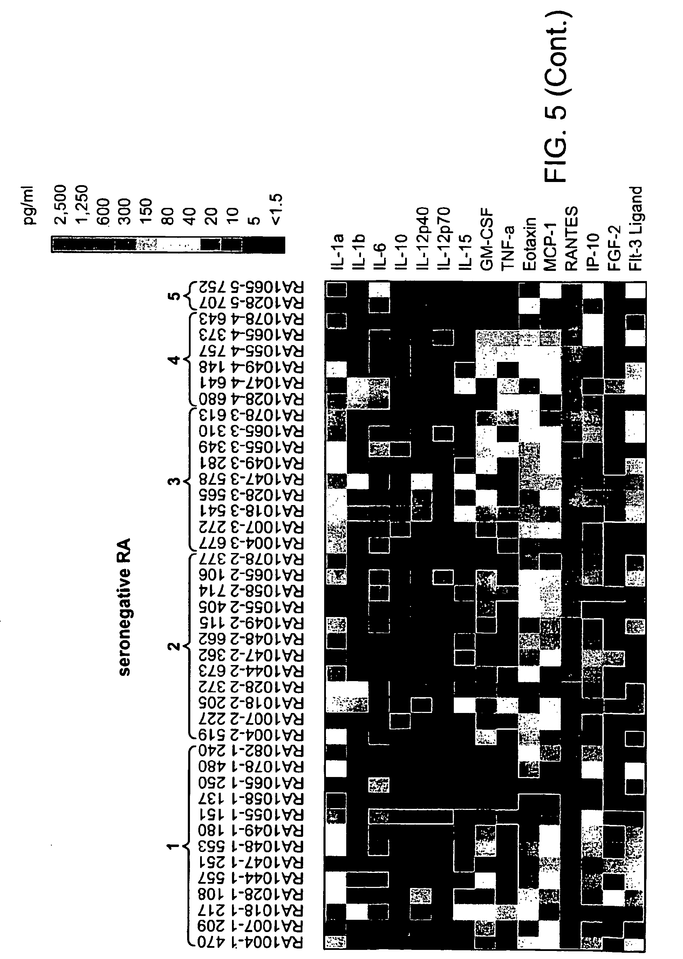

Figure 5. Figure is a heatmap representation of cytokines/chemokines in sero-negative RA patients compared to matched controls. The graph interpreting the color meaning is located in the upper right of the figure, with orange indicating the highest concentration of cytokine/chemokine, and blue representing undetectable levels. The numbers just below the 'controls' and 'seronegative RA' designations refer to the period of time that the serum samples were collected from. Periods 1-4 refer to pre-diagnosis samples, and period 5 contains the post-diagnosis samples (N=2 cases). There were no significant differences in cytokine/chemokine levels in sero-negative RA cases when compared to matched controls.

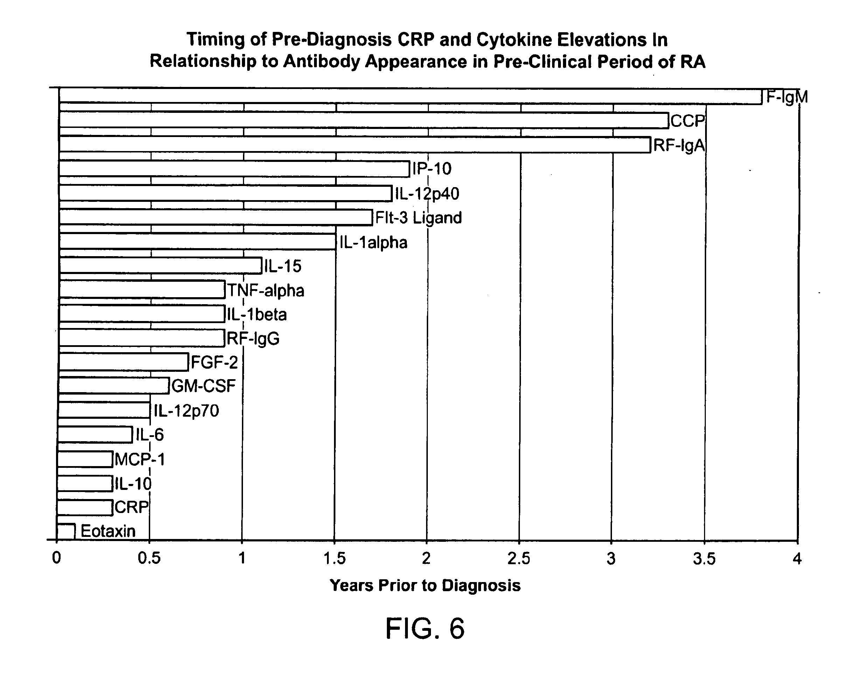

Figure 6. Antibodies precede cytokine/chemokine abnormalities in the pre-clinical period of RA development. Multiplex cytokine analysis on pre-diagnosis stored serum samples from 66 subjects in the U.S. military cohort (Table 3) who developed rheumatoid factor (RF) and/or anti-cyclic citrullinated peptide(anti-CCP) antibody. Samples were tested for c-reactive protein (CRP) and a panel of cytokines/chemokines using a bead-based assay in the presence of HeteroBlock™ to minimize effects of RF. The determination of abnormal levels of cytokines/chemokines and CRP (>0.73 mg/dL) was made using receiver operator curve (ROC) analysis of post-diagnosis RA samples compared to matched controls, with a specificity for RA of >90%. Timing of appearance of antibodies and cytokine/chemokine as well as CRP elevations was determined using interval censored survival analysis (SAS 9.1). RF isotypes IgM, IgA, and IgG appeared a median of 3.8, 3.2, and 0.9 years prior to diagnosis, respectively . Anti-CCP appeared a median of 3.3 years prior to diagnosis. The median times of appearance of the high-specificity (>90%) CRP and the cytokines/chemokine levels tested were within 2 years of diagnosis (Figure 6), although a subgroup including IP-10, IL-12p40, Flt-3 Ligand and IL-1alpha appeared earlier than the others, with a median appearance of >1.5 years.

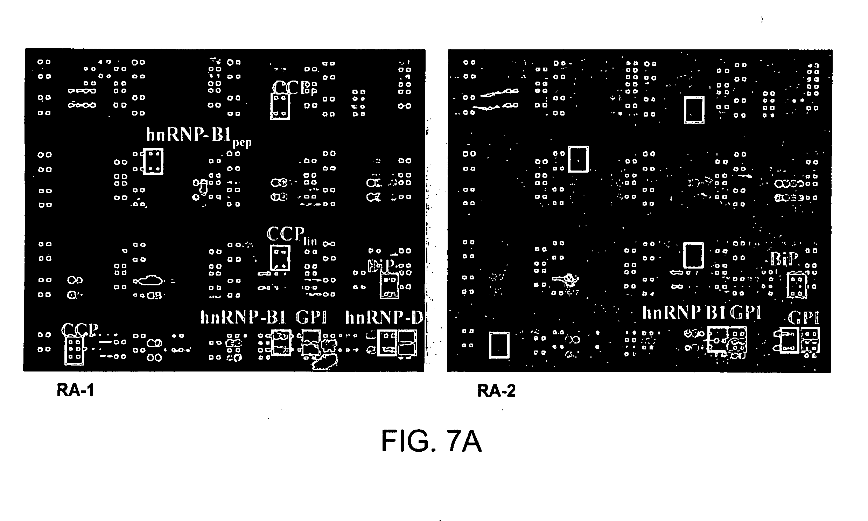

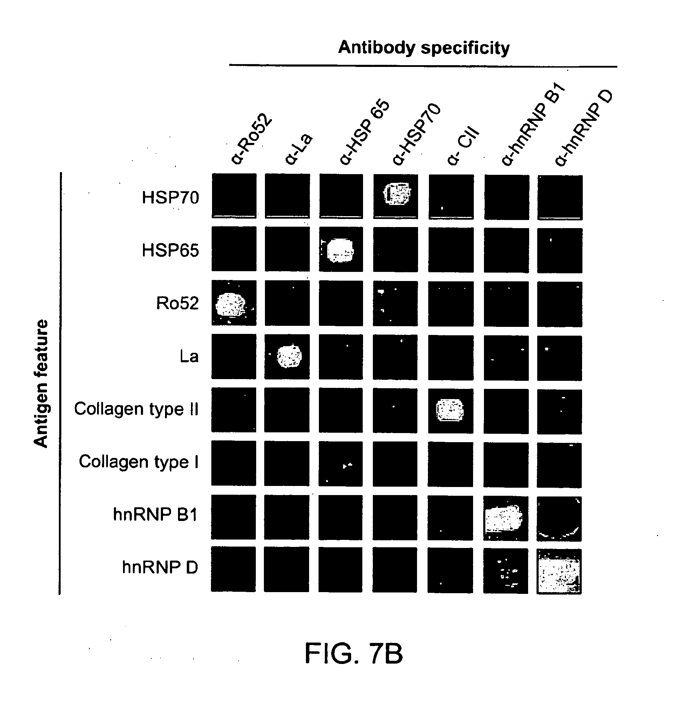

Figure 7. Synovial arrays. (A) Synovial arrays demonstrate heterogeneity of the autoantibody response between RA patients. Arrays were probed with 1:150 dilutions of serum from 2 RA patients. The majority of features are marker features to orient the arrays. RA-1 reacted with CCP1, BiP, hnRNP-B1 and hnRNP-D, while RA-2 reacts with BiP & GPI. (B) Validation of synovial arrays with specific monoclonal antibodies and reference sera.

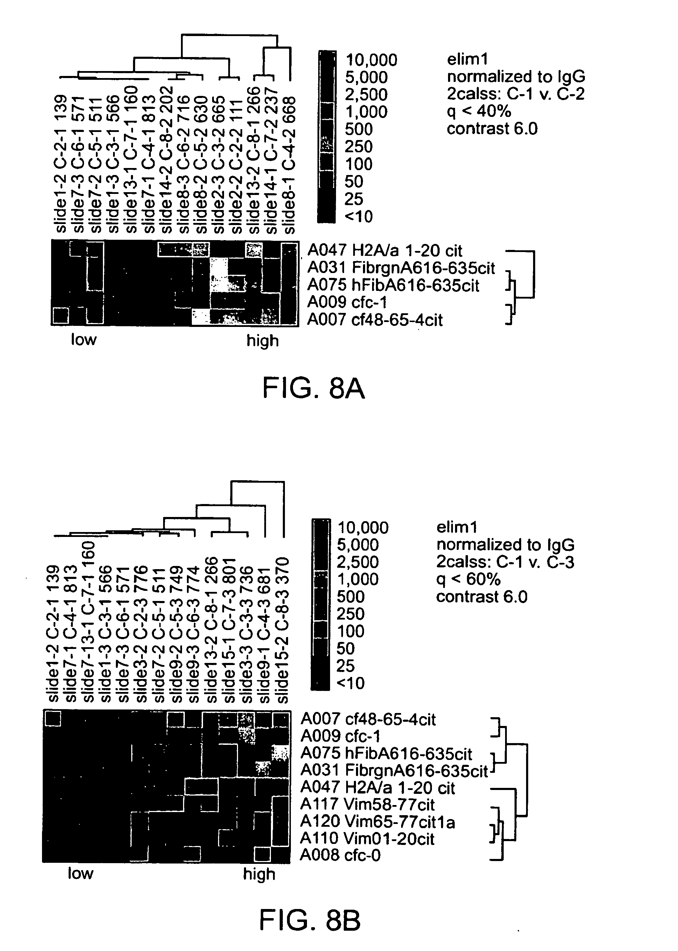

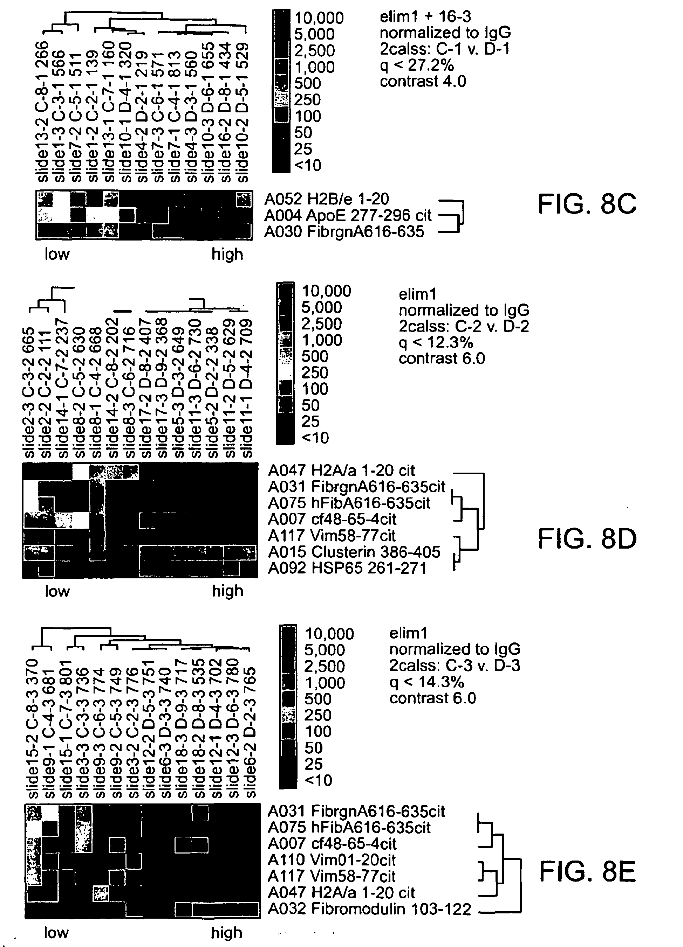

Figure 8. Synovial array analysis demonstrates autoantibody targeting of native and citrullinated epitopes in pre-disease samples derived from RA patients. Synovial peptide arrays containing 130 distinct native or citrulline-substituted peptides were used to profile autoantibodies in serial sera from subjects derived from the described U.S. military pre-disease cohort, obtained at two time points preceding clinical RA (tp1 and tp2) and one time point post diagnosis (tp3). Samples from RA cases were selected for CCP-negative status at tp1 (mean 5.2 yrs. pre-diagnosis), sero-converting to CCP-positive status at tp2 (mean 1.6 yrs. pre-diagnosis) and maintenance of CCP-positive status at tp3 (mean 2.9 yrs. post-diagnosis). Significance analysis of microarrays (SAM) was used to identify differences in relative antibody levels; and hierarchical clustering performed to demonstrate relationships of SAM-identified autoantibodies. Two-class SAM comparisons of pre-symptomatic CCP-negative (tp1) with pre-symptomatic CCP-positive (tp2) serial samples from pre-disease RA patients, demonstrates autoantibody targeting of citrullinated epitopes derived from histone 2A (H2A), fibrinogen (Fibrgn and hFib), and fillagrin (cfc-1 and cf48-65) (A). Comparison of CCP-(tp1) with post-dagnosis (tp3) demonstrated further expansion of autoantibody responses to target additional citrulline-modified epitopes from vimentin (Vim65-77cit and Vim1-20cit). Two-class SAM comparisons of CCP-negative samples with matched controls at tp1 revealed targeting of native epitopes derived from Fibrinogen A and H2B, and the citrullinated epitope ApoE(277-296) cit (C). At tp2, the now CCP-positive samples demonstrated additional targeting of citrullinated epitopes H2A(1-20)cit and Vimentin (58-77)cit, as well as citrullinated Filaggrin peptides (cf48-65cit) (D). At tp3, a similar signature as observed at tp2 was present, now including Vimentin (1-20)cit and N2A(1-20)cit (FDR < 14%) (E). Patients are listed along the top of each heatmap, and the patient number is in the format group-patient#-time point, for example: C-2-1 indicates the RA patient group (group C), patient #2 within that group, and time point 1 (mean 5.2 yr pre-diagnosis); while D-6-2 indicates the matched control group (group D), patient 6 within that group, and time point 2 (mean 1.6 yr pre-diagnosis). The region of the heatmap exhibiting increased autoantibody reactivity is indicated below the heatmap, with "low" indicating low antibody reactivity and "high" indicating elevated antibody reactivity.

DETAILED DESCRIPTION OF THE EMBODIMENTS

The method of claim 1 is provided for prognostic classification of overt RA patients into subtypes, which subtypes are informative of the patient's probability of developing overt disease. The patterns of circulating levels of serum autoantibodies and cytokines identified herein provides for a signature pattern that can discriminate patients who have a high probability of developing overt disease from those who have a low probability of developing overt disease. The signature pattern of autoantibodies and cytokines in a patient has predictive value, and thus allows improved methods of care, where patients can be provided with appropriate therapy.

Various techniques and reagents find use in the diagnostic methods of the present invention. In one embodiment of the invention, blood samples, or samples derived from blood, e.g. plasma, serum, etc. are assayed for the presence of specific cytokines or autoantibodies. Typically a blood sample is drawn, and a derivative product, such as plasma or serum, is tested. Such antibodies may be detected through specific binding members. Various formats find use for such assays, including autoantigen arrays; ELISA and RIA formats; binding of labeled peptides in suspension/solution and detection by flow cytometry, mass spectroscopy, and the like. Cytokine detection may utilize a panel of antibodies specific for a spectrum of cytokines. Autoantibody and cytokine signature patterns typically utilize a detection method coupled with analysis of the results to determine if there is a statistically significant match with a pre-determined signature pattern of interest.

Cytokines may be measured using a panel of antibodies against cytokines, mass spectrometry or with other cytokine detection methods. Panels of anti-cytokine antibodies can be used to measure cytokines in assay formats such as ELISA, fluorescent immunoassays, antibody array technologies, bead array technologies, radioimmunoassay (RIAs) and other immunoassay methodologies.

Mammalian species that provide samples for analysis include canines; felines; equines; bovines; ovines; etc. and primates, particularly humans. Animal models, particularly small mammals, e.g. murine, lagomorpha, etc. may be used for experimental investigations. Animal models of interest include those for models of autoimmunity, graft rejection, and the like.

In some embodiments, the prevalence rates of single and combination cytokine/CRP positivity is determined for pre-diagnosis samples and matched controls. To determine cytokine/chemokine positivity, an AUC analysis is performed with post-diagnosis samples and controls; and a cut-off level is then determined for each cytokine/chemokine that is >90% specific for classification as an RA case. Samples of known disease cases may be divided into pre-diagnosis time intervals and logistic regression analysis performed to determine which of the pre-diagnosis time intervals the biomarkers were most strongly associated with. When analyzed separately both autoantibody positivity and elevations of CRP and cytokines/chemokines are strongly associated with the immediate pre-diagnosis time interval, 0-1 years prior to development of overt disease. The combination of autoantibody panel positivity with CRP or ≥5 cytokine/chemokine positivity provides a strong association with the 0-1 years pre-overt disease diagnosis interval.

Definitions

Terms used in the claims and specification are defined as set forth below unless otherwise specified.

The term "ameliorating" refers to any therapeutically beneficial result in the treatment of a disease state, e.g., an autoimmune disease state, including prophylaxis, lessening in the severity or progression, remission, or cure thereof.

The term "mammal" as used herein includes both humans and non-humans and include but is not limited to humans, non-human primates, canines, felines, murines, bovines, equines, and porcines.

The term "sufficient amount" means an amount sufficient to produce a desired effect, e.g., an amount sufficient to alter a protein expression profile.

The term "therapeutically effective amount" is an amount that is effective to ameliorate a symptom of a disease. A therapeutically effective amount can be a "prophylactically effective amount" as prophylaxis can be considered therapy.

For example, with regard to pre-disease states, one can classify individuals as: normal (N); RF+ anti-CCP- and pre-disease (RFPD); RF+ anti-CCP- not pre-disease (RFNPD); anti-CCP+ RF- pre-disease (CCPPPD), anti-CCP+ RF- not pre-disease (CCPPNPD), RF+ anti-CCP+ pre-disease (RFCCPPPD), and overt RA (RA). Overt RA can be further classified into very early disease (< 6 months of symptoms), early disease (< 2 years of symptoms), and late disease (> 2 years of symptom). Classifications can be modified by the expanded autoantibody profiles as determined by array analysis (both genetic and proteomic), the presence or absence of cytokines, chemokines, non-specific markers of inflammation (CRP, ESR), or genetic risk (ie, presence or absence of certain HLA alleles or susceptibility alleles from genes such as PTPN22 and CTLA-4). Certain characteristics of environmental exposures or answers to standard questionnaires regarding the presence or absence of clinical symptoms may modify risk of overt disease or pre-disease states. Overt disease characterization can include levels of disease activity, degree of joint destruction and disability, and presence or absence of extra-articular features.

Certain classifications are based on validated criteria, for example overt RA is defined by ACR Criteria (Table 1).

Table 1. The 1987 Revised Criteria for the Diagnosis of RA.

| Criterion | Definition |
| --- | --- |
| Morning stiffness | Morning stiffness in and around the joints, lasting at least 1 hour before maximal improvement. |
| Arthritis of 3 or more joint areas | At least three joint areas simultaneously with soft tissue swelling or joint fluid observed by a physician; the 14 possible areas are (right or left): PIP, MCP, wrist, elbow, knee, ankle, and MTP joints. |
| Arthritis of hand joints | At least 1 area swollen in a wrist, MCP, or PIP joint |
| Symmetric arthritis | Simultaneous involvement of the same joint areas on both sides of the body (bilateral involvement of the PIP, MCP, or MTP acceptable without perfect symmetry). |
| Rheumatoid nodules | Subcutaneous nodules over bony prominences or extensor surfaces, or in juxtaarticular regions, observed by a physician. |
| Serum rheumatoid factor | Abnormal amount of serum RF by any method for which the result has been positive in <5% of control subjects |

(continued)

| Criterion | Definition |
|---|---|
| Radiographic changes | Erosions or unequivocal bony decalcification localized in or most marked adjacent to the involved joints (osteoarthritis changes excluded), typical of RA on posteroanterior hand and wrist radiographs. |

| For classification purposes, a patient is said to have RA if four of seven criteria are present. Criteria 1-4 must have been present for at least 6 weeks. *Taken from Arnett FC, Edworthy SM, Bloch DA, McShane DJ, Fries JF, Cooper NS  Healey LA, Kaplan SR, Liang MH, Luthra HS, et al. The American Rheumatism Association 1987 revised criteria for the classification of rheumatoid arthritis. Arthritis Rheum 1988;31:315-324. |
|---|

Analysis of results may utilize the common meaning certain terms, including:

TP: true positive
TN: true negative
FP: false positive
FN: false negative
N: total number of negative samples
P: total number of positive samples
A: total number of samples

$$Accuracy = (TP+TN)/A$$

Mean CV error = Mean Misclassification error = 1- Mean Accuracy

$$Sensitivity = TP/P = TP/(TP+FN)$$

$$Specificity = TN/N = TN/(TN+FP)$$

It must be noted that, as used in the specification and the appended claims, the singular forms "a," "an," and "the" include plural referents unless the context clearly dictates otherwise.

Several features of the current approach should be noted. Autoimmune disease and related conditions are diagnosed through a blood based test that assesses the presence of a panel of protein markers as defined in the claims. In some embodiments, such a predictive model utilizes quantitative data obtained from circulating markers that further include RF antibodies and anti-CCP antibodies, and may further include a variety of additional markers as described herein, including clinical indicia, metabolic measures, genetic assays, and additional circulating markers. In a preferred embodiment the dataset comprises quantitative data for the group consisting of eotaxin (CCL11), IL-lalpha, GM-CSF, MCP-1 (CCL2), and IP-10 (CXCL-10).

In another embodiment of the invention, at least two, at least three, at least four, at least five or more markers are selected from IP-10 (CXCL-10), MCP-1, CRP, eotaxin, GM-CSF, FGF-2, Flt-3 ligand, IL-10, IL-12 p40, IL-12 p70, IL-15, IL-1$\alpha$, IL-10, IL-6, and TNF$\alpha$.

The identification of autoimmune associated circulating proteins provides prognostic methods, which assess an individual's susceptibility to such disease, by detecting altered levels of the identified circulating proteins. Early detection can be used to determine the occurrence of developing disease, thereby allowing for intervention with appropriate preventive or protective measures.

Table 2. Circulating proteins of interest.

| Protein | Human polynucleotide accession | Human protein accession |
|---|---|---|
| IP-10 (CXCL-10) | NM_001565 | NP_001556.1 |
| MCP-1 | NM_002982 | NP_002973.1 |
| CRP | NM_000567 | NP 000558.2 |
| eotaxin | NM_005408 | NM_005408 |

(continued)

| Protein | Human polynucleotide accession | Human protein accession |
| --- | --- | --- |
| GM-CSF | M11220.1 | P04141 |
| FGF-2 | NM_002006.2 | NP_001997 |
| Flt-3ligand | NM_001459.2 | g38455416 |
| IL10 | NM_000572 | NP 000563 |
| IL12 p40 | NM_002187 | NP_002178 |
| IL12 p70 | NM_000882.2 | NP_445842.1 |
| IL-15 | U14407.1 | NP_000576.1 |
| IL-1α | NM_000575.3 | P01583 |
| IL1β | NM_000576 | NP_000567 |
| IL6 | NM_000600 | NP_000591 |
| TNFα | NM_000594 | NP_000585 |

In addition to the specific biomarker sequences identified in this application by name, accession number, or sequence, the invention also contemplates use of biomarker variants that are at least 90% or at least 95% or at least 97% identical to the exemplified sequences and that are now known or later discover and that have utility for the methods of the invention. These variants may represent polymorphisms, splice variants, mutations, and the like. Various techniques and reagents find use in the diagnostic methods of the present invention. In one embodiment of the invention, blood samples, or samples derived from blood, e.g. plasma, circulating, etc. are assayed for the presence of polypeptides. Typically a blood sample is drawn, and a derivative product, such as plasma or serum, is tested. Such polypeptides may be detected through specific binding members. The use of antibodies for this purpose is of particular interest. Various formats find use for such assays, including antibody arrays; ELISA and RIA formats; binding of labeled antibodies in suspension/solution and detection by flow cytometry, mass spectroscopy, and the like. Detection may utilize one or a panel of antibodies, preferably a panel of antibodies in an array format. Expression signatures typically utilize a detection method coupled with analysis of the results to determine if there is a statistically significant match with a disease signature.

The cytokine analysis further utilizes analysis of antibodies specific for autoantigens as defined in claim 1. Antigens may further include molecules such as nucleic acids, lipids, ribonucleoprotein complexes, protein complexes, proteins, polypeptides, peptides and naturally occurring or synthetic (in vitro) modifications of such molecules against which an immune response involving T and B lymphocytes can be generated. For each antigen, there exists a panel of epitopes that represent the immunologic determinants of that antigen. Antigens include any molecule that can be recognized, all or in part, by an antibody or T cell receptor.

Autoantigens are any molecule produced by the organism that are the target of an immunologic response, including lipids, nucleic acids, peptides, polypeptides, and proteins encoded within the genome of the organism. Such molecule also include post-translationally-generated modifications of these peptides, polypeptides, and proteins, such as cleavage, phosphorylation, deimination of arginine to citrulline, and other modifications generated through physiologic and non-physiologic cellular processes. Such molecules also include modifications of these biomolecules, such as oxidation, cleavage products, and degradation products.

Epitopes are portions of antigens that are recognized by antibodies or T cell antigen receptors. An individual antigen typically contains multiple epitopes, although there are instances in which an antigen contains a single epitope. In one embodiment of this invention, peptide fragments derived from a whole protein antigen are used to represent individual epitope(s) targeted by the antibodies produced by B cells. In another embodiment, portions of molecules representing post-translational modifications, carbohydrates, lipids and other molecules can be used to represent individual epitopes. Epitopes represent shapes recognized by immune B and T cells, and can also be represented by non-antigen derived peptides and other molecules that possess the same epitope shape that is present within the native antigen. An example of an element with an epitope shape is an aptamer. An aptamer is a molecule that provides a shape that can mimic an immunologic epitope. Using a plurality of aptamers a library of epitope shapes can be generated. Where peptides are used as an epitope to detect antibody binding, peptides will usually be at least about 7 amino acids in length, may be at least about 15 amino acids in length, and as many as 22 amino acids in length. The peptides of a protein may be overlapping by 7-10 amino acids, and can encompass the whole sequence of a protein of interest.

For the prognosis of rheumatoid arthritis, the detection of rheumatoid factor (RF) and anti-CCP are of interest. RF is an autoantibody specific for immunoglobulin Fc regions. High levels RF (generally above 20 IU/mL, 1:40 or over the 95th percentile) can be indicative of rheumatoid arthritis. Rheumatoid factors can be detected using the technique of nephelometry as well as specific ELISA or other assays for IgM, IgG or IgA isotypes. The presence or absence of specific RF tests can modify the risk of disease or pre-disease states. Anti-CCP is an antibody directed against a circular peptide containing citrulline. The anti-CCP appears early in the course of rheumatoid arthritis and is present in the blood of most patients with the disease.

*Rheumatoid Arthritis* is a chronic syndrome characterized by usually symmetric inflammation of the peripheral joints, potentially resulting in progressive destruction of articular and periarticular structures, with or without generalized manifestations. The cause is unknown. A genetic predisposition has been identified and, in white populations, localized to a pentapeptide in the HLA-DR beta1 locus of class II histocompatibility genes. Additional non-HLA genes are also associated with increased risk for disease including polymorphisms in PTPN22 and CTLA4 loci. Environmental factors may also play a role. Immunologic changes may be initiated by multiple factors. About 0.6% of all populations are affected, women two to three times more often than men. Onset may be at any age, most often between 25 and 50 yr. In over 80% of cases, RA is characterized by the presence of the autoantibodies rheumatoid factor (RF) and antibodies directed against citrullinated peptides, of which the antibody to a cyclic citrullinated peptide (anti-CCP antibody) is most clinically available. These antibodies have been shown to present years prior to the onset of symptoms in RA, suggesting that the immunologic processes that lead to disease are present long before overt disease manifestations (Nielen MM, van Schaardenburg D, Reesink HW, van de Stadt RJ, van der Horst-Bruinsma IE, de Koning MH, et al. Specific autantibodies precede the symptoms of rheumatoid arthritis: a study of serial measurements in blood donors. Arthritis Rheum. 2004 Feb;50(2):380-6; Rantapaa-Dahlqvist S, de Jong BA, Berglin E, Hallmans G, Wadell G, Stenlund H, et al. Antibodies against cyclic citrullinated peptide and IgA rheumatoid factor predict the development of rheumatoid arthritis. Arthritis Rheum. 2003 Oct;48(10):2741-9. Also, in additional pre-clinical studies the presence of specific HLA DR4 alleles along with RA-specific antibodies was highly predictive of eventual development of symptomatic RA (Berglin E, Padyukov L, Sundin U, Hallmans G, Stenlund H, Van Venrooij WJ, Klareskog L, Dahlqvist SR. A combination of autoantibodies to cyclic citrullinated peptide (CCP) and HLA-DRB1 locus antigens is strongly associated with future onset of rheumatoid arthritis. Arthritis Res Ther. 2004;6(4):R303-8. Epub 2004 May 11). RA that does not exhibit autoantibodies is termed sero-negative RA and comprises less than 20% of the total cases. This variation of disease represents a distinct subset that appears both immunolgically and clinically different that sero-positive RA.

Prominent immunologic abnormalities that may be important in pathogenesis include immune complexes found in joint fluid cells and in vasculitic lesions. Plasma cells produce antibodies that contribute to these complexes. Lymphocytes that infiltrate the synovial tissue are primarily T helper cells, which can produce pro-inflammatory cytokines. Macrophages and their cytokines (e.g., tumor necrosis factor, granulocyte-macrophage colony-stimulating factor) are also abundant in diseased synovium. Increased adhesion molecules contribute to inflammatory cell emigration and retention in the synovial tissue. Increased macrophage-derived lining cells are prominent along with some lymphocytes and vascular changes in early disease. Effector molecules such as complement activation fragments are released into the tissue and joint fluid and can cause both direct and indirect injury. Engagement of activating Fc receptors by immune complexes can potentiate injury to the joint.

In chronically affected joints, the normally delicate synovium develops many villous folds and thickens because of increased numbers and size of synovial lining cells and colonization by lymphocytes and plasma cells. The lining cells produce various materials, including collagenase and stromelysin, which can contribute to cartilage destruction; interleukin-1, which stimulates lymphocyte proliferation; and prostaglandins. The infiltrating cells, initially perivenular but later forming lymphoid follicles with germinal centers, synthesize interleukin-2, other cytokines, RF, and other immunoglobulins. Fibrin deposition, fibrosis, and necrosis also are present. Hyperplastic synovial tissue (pannus) may erode cartilage, subchondral bone, articular capsule, and ligaments. PMNs are not prominent in the synovium but often predominate in the synovial fluid.

Onset of clinically apparent disease is usually insidious, with progressive joint involvement, but may be abrupt, with simultaneous inflammation in multiple joints. Tenderness in nearly all inflamed joints is the most sensitive physical finding. Synovial thickening, the most specific physical finding, eventually occurs in most involved joints. Symmetric involvement of small hand joints (especially proximal interphalangeal and metacarpophalangeal), foot joints (metatarsophalangeal), wrists, elbows, and ankles is typical, but initial manifestations may occur in any joint.

In RA, response to therapy is conventionally measured using the American College of Rheumatology (ACR) Criteria. The ACR response criteria are a composite score comprising clinical (swollen joint count, tender joint count, physician and patient response assessment, and health assessment questionnaire), and laboratory (acute phase response) parameters; level of improvement is reported as an ACR20 (20%), ACR50 (50%) or ACR70 (70%) response, which indicates percent change (improvement) from the baseline score. A number of clinical trails based on which the anti-TNFα agents infliximab (Remicade™), etanercept (Enbrel™) and adalimumab (Humira™) were approved to treat human RA utilized ACR response rates as a primary outcome measure.

Responses in rheumatoid arthritis many also be assessed using other response criteria, such as the Disease Activity Score (DAS), which takes into account both the degree of improvement and the patient's current situation. The DAS has been shown to be comparable in validity to the ACR response criteria in clinical trials. The definitions of satisfactory and unsatisfactory response, in accordance with the original DAS and DAS28. The DAS28 is an index consisting of a 28 tender joint count, a 28 swollen joint count, ESR (or CRP), and an optional general health assessment on a visual analogue scale (range 0-100) (Clinical and Experimental Rheumatology, 23(Suppl. 39):S93-99, 2005). DAS28 scores are being used for quantification of response mostly in European trials of (early) rheumatoid arthritis such as the COBRA or BeST studies (described below). In asymptomatic individuals predicted to develop RA, response could be assessed based on progression to clinically definite RA based on the 1987 Revised Criteria for the Diagnosis of RA (Table 1).

Radiographic measures for response in RA include both conventional X-rays (plain films), and more recently magnetic resonance (MR) imaging, computed tomography (CT), ultrasound and other imaging modalities are being utilized to monitor RA patients for disease progression. Such techniques are used to evaluate patients for inflammatoin (synovitis), joint effusions, cartilage damage, bony erosions and other evidence of joint damage. Methotrexate, anti-TNF agents and DMARD combinations have been demonstrated to reduce development of bony erosions and other measures of joint inflammation and destruction in RA patients. In certain cases, such as with anti-TNF agents, healing of bony erosions has been observed.

THERAPEUTIC AGENTS (not claimed)

General classes of drugs commonly used in the non-antigen specific treatment of autoimmune disease include corticosteroids and disease modifying drugs. Corticosteroids have a short onset of action, but many disease modifying drugs take several weeks or months to demonstrate a clinical effect. These agents include methotrexate, leflunomide (Arava™), etanercept (Enbrel™), infliximab (Remicade™), adalimumab (Humira™), anakinra (Kineret™), rituximab (Rituxan™), CTLA4-Ig (abatacept), antimalarials, gold salts, sulfasalazine, d-penicillamine, cyclosporin A, cyclophosphamide azathioprine; and the like.

Corticosteroids, e.g. prednisone, methylpredisone, prednisolone, solumedrol, etc. have both anti-inflammatory and immunoregulatory activity. They can be given systemically or can be injected locally. Corticosteroids are useful in early disease as temporary adjunctive therapy while waiting for disease modifying agents to exert their effects. Corticosteroids are also useful as chronic adjunctive therapy in patients with severe disease that is not well controlled on NSAIDs and other agents.

Disease modifying anti-rheumatoid drugs, or DMARDs have been shown to alter the disease course and improve radiographic outcomes. It will be understood by those of skill in the art that these drugs are also used in the treatment of other autoimmune diseases. DMARDs have an effect upon rheumatoid arthritis that is different and more delayed in onset than NSAIDs, corticosteroids, or antigen specific therapies.

Methotrexate has become a popular first-line agent in RA and other autoimmune diseases because of its early onset of action (4-6 weeks), good efficacy, favorable toxicity profile, ease of administration, and relatively low cost. Methotrexate is the only conventional DMARD agent in which the majority of patients continue on therapy after 5 years. Methotrexate is effective in reducing the signs and symptoms of RA, as well as slowing or halting radiographic damage. Although the immunosuppressive and cytotoxic effects of methotrexate are due to the inhibition of dihydrofolate reductase, the anti-inflammatory effects in rheumatoid arthritis appear to be related at least in part to interruption of adenosine and TNF pathways. The onset of action is 4 to 6 weeks, with 70% of patients having some response. A trial of 3 to 6 months is suggested.

Antimalarials such as hydroxychloroquine and chloroquine are rapidly absorbed, relatively safe, well-tolerated and often effective remittive agents for the treatment of rheumatoid arthritis, particularly mild to moderate disease. Hydroxychloroquine (Plaquenil, 200mg tablets) is the drug of choice among antimalarials. The usual dose is 400mg/day (6mg/kg) but 600mg/day is sometimes used. Normally it is prescribed as a single nighttime dose to avoid gastrointestinal symptoms. A period of 2 to 4 months is usual to take effect. A 6-month period without clinical effect should be considered a drug failure.

Sulfasalazine is another effective DMARD for the treatment of RA. Its mechanism of action in RA is unknown. Like the other DMARDs, it has been shown not only to reduce the signs and symptoms of RA but also to slow or halt radiographic progression. It can cause hypersensitivity reactions due to sulfa allergy, mild gastrointestinal, and occasionally, mild cytopenias. The usual dose is 2-3 grams per day in a twice daily dosing regimen. Blood monitoring is every 1-3 months depending on dose. Sulfasalazine is a good alternative to methotrexate for patients with liver disease.

Leflunomide (Arava™) was approved by the FDA and became available as a new DMARD agent for rheumatoid arthritis in October 1998. In clinical trials, its efficacy was similar to that of methotrexate and it represents a viable alternative to patients who have failed or are intolerant to methotrexate. Leflunomide has been demonstrated to slow radiographic progression and damage in RA. It can also be combined with methotrexate in patients with no preexisting liver disease, as long as the liver function tests are carefully monitored. The mechanism of action of leflunomide is not fully understood but may be related to its ability to inhibit tyrosine kinase activity and inhibit *de novo* pyrimidine biosynthesis through the inhibition of the enzyme dihydroorotate dehydrogenase. *In vitro* studies have demonstrated the inhibition of mitogen and IL-2 stimulated T cells. To achieve steady state, a loading dose of 100mg daily for three days can be given followed by 20 mg daily. However, more recent recommendations are for a starting dose of 20mg daily. The dose may be reduced to 10mg daily if not tolerated or in patients having difficulty metabolizing or excreting the drug. Onset of action is in 4-8 weeks.

Tumor necrosis factor alpha (TNF-α) is a pro-inflammatory cytokine produced by macrophages and lymphocytes. It is found in large quantities in the rheumatoid joint and is produced locally in the joint by synovial macrophages and lymphocytes infiltrating the joint synovium. Extensive clinical trial data have confirmed the efficacy of all three currently available TNF inhibitors in relieving the signs and symptoms of RA, and in slowing or halting radiographic damage. The strategies for inhibiting TNF that have been most extensively studied to date consist of monoclonal anti-TNF antibodies and soluble TNF receptors (sTNF-R). Both constructs will bind to circulating TNF-α, thus limiting its ability to engage cell membrane-bound TNF receptors and activate inflammatory pathways. Soluble TNF-R, but not anti-TNF antibodies, also bind lymphotoxin.

One of the monoclonal anti-TNF antibodies is infliximab (Remicade®), originally referred to as cA2. Infliximab is a chimeric human/mouse monoclonal anti-TNFα antibody composed of the constant regions of human (Hu) IgG1κ, coupled to the Fv region of a high-affinity neutralizing murine anti-HuTNFα antibody. The antibody exhibits high affinity (Ka 1010/mol) for recombinant and natural huTNFα, and neutralizes TNF-mediated cytotoxicity and other functions *in vitro.* An alternate strategy has been to develop a fully human anti-TNF monoclonal antibody. One such antibody, known as D2E7, also known as adalumimab, was generated by phage display technology. A high affinity murine anti-TNF monoclonal antibody was used as a template for guided selection, which involves complete replacement of the murine heavy and light chains with human counterparts and subsequent optimization of the antigen-binding affinity. D2E7 (adalimumab, Humira™) received FDA approval in December, 2002.

Alternatively, soluble TNF-R have been engineered as fusion proteins in which the extracellular ligand-binding portion of the huTNF-RI or huTNF-RII is coupled to a human immunoglobulin-like molecule. Although TNF-RI is thought to mediate most of the biological effects of TNF *in vivo,* engineered sTNF-RI and sTNF-RII constructs both appear to be effective *in vivo* inhibitors of TNF. Etanercept (sTNF-RII:Fc; Enbrel™) is the best studied of the sTNF-R and is approved for the treatment of rheumatoid arthritis in adults and in children. It is a dimeric construct in which two sTNF-RII (p75) are linked to the Fc portion of human IgG1. The dimeric receptor has a significantly higher affinity for TNF-α than the monomeric receptor (50-1000-fold higher), and the linkage to the Fc structure significantly prolongs the half-life of the construct *in vivo.* Although it also has an unnatural linkage site, anti-etanercept antibodies have been infrequent. Another mechanism for prolonging the half-life of monomeric receptors is via conjugation with polyethylene glycol. One such construct, PEG-sTNF-RI (p55), has shown efficacy in several animal models of arthritis and is now in early clinical trials.

It is well established that approximately 1/3 of patients exhibit a robust clinical response following initiation of any one of the 3 FDA-approved anti-TNF therapies (etanercept, adalimumab and remicade) (Doan, O.V., et al, (2006) J Manag Care Pharm. 12(7):555-69). As described, clinical response is measured based on the American College of Rheumatology (ACR) response criteria, and the 1/3 of patients that are experience robust clinical responses experience an ACR50 or greater response. A second 1/3 of patients experience a partial response to any one of the FDA approved agents, approximately an ACR20 response. The remaining 1/3 of RA patients exhibit no meaningful clinical response when initiated on an approved anti-TNF therapy. There is great clinical need for biomarkers to identify RA patients, or which asymptomatic patients predicted to develop RA, will likely to respond vs. not respond to an ant-TNF agent given: (1) the potentially serious side effects of anti-TNF agents including (a) activation of tuberculosis, (b) increased rates of serious and life threatening infections, and (c) increased rates of demyelinating lesions; (2) the significant expense of anti-TNF therapies (approximately $15,000 USD per year of therapy), and (3) the availability of multiple other potential effective small molecule and biological agents (methotrexate, leuflonamide, anakinra, CTLA4-Ig).

Soluble Interleukin-1 (IL-1) Receptor therapy. IL-1 is a cytokine that has immune and pro-inflammatory actions and has the ability to regulate its own expression by autoinduction. Evidence supports the fact that the level of disease activity in RA, and progression of joint destruction, correlate with plasma and synovial fluid levels of IL-1. IL-1ra is an endogenous receptor antagonist. Evidence supporting the anti-inflammatory role of IL-1ra *in vivo* is demonstrated by the observation that IL-1ra deficient mice spontaneously develop autoimmune diseases similar to rheumatoid arthritis and arteriitis.

Anakinra (Kineret™) is a human recombinant IL-1 receptor antagonist (hu rIL-1ra) approved by the FDA for the treatment of RA. Anakinra can be used alone or in combination with DMARDs other than TNF blocking agents (Etanercept, Infliximab). Anakinra is a recombinant, nonglycosylated form of the human IL-1ra. It differs from the native nonglycosylated IL-1ra by the addition of an N-terminal methionine. Anakinra blocks the biologic activity of IL-1 by binding to IL-1R type I with the same affinity as IL-1β. Usual time to effect is 2 to 4 weeks.

Cytotoxic T lymphocyte-associated antigen 4 (CTLA4) is an immunoregulatory protein expressed on the T cell surface after activation. It binds to CD80 or CD86, blocks their interaction with CD28, and thus acts as an off-switch for cell activation. CTLA4Ig is a genetically engineered fusion protein that consists of a human CTLA4 portion fused to a constant IgG1 region (also know as Abetacept, produced by Bristol-Myers Squib, New York City, New York, USA). This molecule binds to CD80 and CD86 and thereby inhibits T cell co-stimulation. Abetacept was approved by the US Food and Drug Administration for the treatment of RA.

Rituximab. The CD20 antigen is present on the cell surface of all pre-plasma cell stages of B cell differentiation.

The mature plasma cell loses the CD20 antigen, and thus it serves as a relatively specific marker for B cells. Rituximab (Roche Pharmaceuticals, Basel, Switzerland; Genentech, South San Francisco, USA; IDEC Pharmaceuticals, San Diego, USA), a genetically engineered human-mouse chimeric monoclonal antibody against the CD20 antigen, binds to the CD20 antigen on the B cell surface and efficiently depletes B cells by antibody-dependent and complement-dependent cell lysis.

The most commonly used cytotoxic drugs are azathioprine (Imuran), cyclophosphamide (Cytoxan) and cyclosporin A. Because the potential of high toxicity, these agents are utilized for life-threatening extra-articular manifestations such as systemic vasculitis or severe articular disease refractory to other therapy. It is recommended that these agents be used under the direction of a rheumatologist. Azathioprine is a purine analog. Cyclophosphamide is an alkylating agent. Cyclosporine is an immunosuppressive agent approved for use in preventing renal and liver allograft rejection. Cyclosporine inhibits T cell function by inhibiting transcription of interleukin-2. Main toxicities include infection and renal insufficiency.

Interleukin-6 is a glycoprotein composed of 184 amino acids. Numerous cells can produce this inducible cytokine, including macrophages, B cells, T cells, fibroblasts, endothelial cells, mesangial cells, and many types of tumor cells. The effects of IL-6 are pleiotropic, occurring at both a systemic and a local tissue level, and involving a wide variety of cells. Of particular relevance to RA are the effects on the differentiation of B and T lymphocytes, as well as the differentiation of macrophages, megakaryocytes, and osteoclasts. Interleukin-6 is elevated in the serum and synovial fluid in RA patients. The excessive production of IL-6 is postulated to play a role in the pathogenesis of several inflammatory diseases such as RA, Crohn's disease, and juvenile idiopathic arthritis. In RA, IL-6 participates in immune cell activation and autoantibody production, osteoclastogenesis, and bone loss, and the often debilitating systemic and constitutional symptoms associated with the acute-phase response. MRA (Chugai Pharmaceutical Co. Ltd., Tokyo, Japan) is a humanized anti-IL-6 receptor antibody (Tocilizumab) that inhibits the binding of IL-6 to its receptor IL-6R and prevents IL-6 signal transduction.

Trials targeting other cytokines, including IL-12, IL-15, and IL-18 are under way. AMG 714 (Genmab, Copenhagen, Denmark) is a human monoclonal antibody that binds to IL-15 and inhibits its signaling. Patients receiving AMG 714 had clinically meaningful improvement compared with placebo, demonstrating that IL-15 is a target in the treatment of RA. In preclinical studies, an anti-IL-17 antibody significantly reduced the severity of collagen-induced arthritis. BlyS, or BAFF, is a member of the tumor necrosis factor family of cytokines, and its receptors, BCMA, BAFFR, and TACI, are largely restricted to B cells (a small amount of TACI has been found on activated T cells). LymphoStat-B is a fully human IgG1λ monoclonal antibody that neutralizes human BlyS. The administration of LymphoStat-B to cynomolgus monkeys selectively reduces B cells in blood and tissue with no overt toxicity.

There is growing evidence that early and aggressive treatment of a variety of pre-symptomatic or early disease states can reduce the development, severity and/or progression of autoimmune disease. For example, treatment of recently diagnosed (< 2 years) RA patients with combination therapy with methotrexate, sulfasalazine and/or prednisone or with methotrexate and infliximab, with tapering to monotherapy once low disease activity was achieved, resulted in a significant number of patients achieving remission (Clin Exp Rheumatol. 2006 Nov-Dec;24(6 Suppl 43):S077-82.). Following 2 years of therapy, 80% of patients exhibited a Disease Activity Score (DAS) of <= 2.4, and 42% reached clinical remission as defined by DAS < 1.6. Patients in this study were also monitored using the Health Assessment Questionnaire (HAQ) and hand radiographs. Based on this study, the authors conclude that in recent onset RA, significant clinical benefit and suppression of joint damage can be achieved through combination therapy with methotrexate, sulphasalazine and prednisone or therapy with methotrexate and infliximab, and that combination therapy can be reduced to monotherapy following achievement of low disease activity (Clin Exp Rheumatol. 2006;24(6 Suppl 43):S077-82.)

A recent study utilizing methotrexate in early undifferentiated inflammatory arthritis showed that fulfillment of full ACR criteria for RA as well as delayed onset of erosions is possible with early, aggressive therapy (van Dongen H, van Aken J, Lard JR, et al. Efficacy of methotrexate treatment in patients with probable rheumatoid arthritis: a double-blind, randomized, placebo-controlled trial. Arthritis Rheum 2007;56(5):1424-1432.. In addition, use of high-dose corticosteroids early in RA has been shown to result in increased rates of remission (Green M, Marzo-Ortega H, McGonagle D, et al. Persistence of mild, early inflammatory arthritis: the importance of disease duration, rheumatoid factor, and the shared epitope. Arthritis Rheum 1999;42(10):2184-8).

Based on these studies, it is likely that even earlier aggressive treatment could provide greater efficacy in reducing the clinical incidence of RA and/or disease severity. It is anticipated that treatment of patients at the time of diagnosis of RA, at the time of initial development of clinical arthritis (4-6 months prior to the time at which RA can be diagnosed), and/or during the asymptomatic pre-clinical period could provide significant benefit. It is also anticipated that treatment of such patients with monotherapies or combination therapies including methotrexate, leuflonamine, anti-TNF agents, CTLA4-Ig, anti-IL-6, rituximab and/or other small molecule or biological therapies could significantly reduced the incidence and/or severity of RA.

Also of concern is the potential for overtreatment of the subset of early arthritis patients who will experience a benign disease course. It is well established that a subset of early arthritis patients, including patients with early RA, will experience spontaneous natural remission in the absence of therapeutic intervention. Thus, biomarkers are needed to identify and differentiate such patients from patients who will develop full-blow and/or severe RA. Patients predicted to have benign and naturally remitting RA would likely be treated with NSAIDs and other "low-impact" therapies, while patients predicted to evolve to established RA would be treated more aggressively with DMARD therapy, and patients predicted to develop severe debilitating RA would be treated most aggressively with highly potent DMARD therapy. Such a therapeutic strategy could both reduce the incidence of RA, by reducing the number of patients that progress from early arthritis or RA to established RA, as well as reduce the mortality and morbidity from RA.

Thus, analysis of biomarkers, including individual and/or combinations of antibodies, cytokines, inflammatory markers, and genetic markers will facilitate identification of individuals likely to develop RA, with early RA, or more likely to experience a more severe clinical course. In certain cases, combination of such biomarkers with clinical features, standards laboratory test results, and/or resulting from radiographic or other imaging studies, could provide additional predictive value for identifying patients with a high likelihood of developing RA, or with early RA, or with severe RA. Early intervention with MTX, anti-TNF, sulfasalazine, and/or other biological agents will likely reduce the rate of progression to clinically definite RA, or in the case of patients with early or severe RA reduce the progression and severity of RA.

Clinically isolated synodrome (CIS) is the development of a neurologic deficit such as optic neuritis (blurry vision), motor weakness (in an arm or a leg), sensory deficits (numbness), gait instability (difficulty walking due to cerbellar or other neurologic dysfunction). A subset of patients with CIS will progress to clinically definite MS based on development of a second independent neurlogic deficit. In patients with optic neuritis and >2 demyelinating lesions on magnetic resonance imaging of the brain, treatment with a short-term high dose corticosteroids (1g methyprednisolone IV daily for 3 days) reduced the development of clinically definite MS by 50% at 2 years (N Engl J Med. 1993;329(24):1764-9.). In other studies, treatment with interferon beta reduced progression to clinically definite MS in patients with CIS who exhibited subclinical demyelination on magnetic resonance imaging of the brain (Neurol Sci. 2003 Dec;24 Suppl 5: S298-300).

Thus, biomarkers that facilitate identification of asymptomatic individuals likely to develop an autoimmune disease, or early symptomatic individuals likely to progress to an autoimmune disease, will facilitate implementation of therapies that could reduce the ultimate incidence and/or severity of clinically active disease. Such biomarkers may identify patients likely to experience a mild disease course and thus warrant less aggressive therapy from patients likely to develop severe disease and thus warrant more aggressive therapy. Further, such biomarkers may identify patients likely to respond to specific therapeutic agents, and could thereby be used to guide selection of the most appropriate agent(s) to treat individual patients that are asymptomatic (pre-disease), exhibiting early symptoms, or have established disease.

DIAGNOSTIC AND PROGNOSTIC METHODS

The differential presence of cytokines, and specific autoantibodies is shown to provide for prognostic evaluations to detect individuals in a pre-disease state. In general, such prognostic methods involve determining the presence or level of cytokines in an individual sample, usually a blood derived sample, e.g. blood, serum, plasma, etc. A variety of different assays can be utilized to quantitate the presence of cytokines. Many such methods are known to one of skill in the art, including ELISA, fluorescence immunoassays, protein arrays, eTag system, bead based systems, tag or other array based systems, surface plasmon resonance (SPR)-based detection systems, etc. Examples of such methods are set forth in the art, including, inter alia, chip-based capillary electrophoresis: Colyer et al. (1997) J Chromatogr A. 781 (1-2):271-6; mass spectroscopy: Petricoin et al. (2002) Lancet 359: 572-77; eTag systems: Chan-Hui et a/. (2004) Clinical Immunology 111:162-174; microparticle-enhanced nephelometric immunoassay: Montagne et a/. (1992) Eur J Clin Chem Clin Biochem. 30(4):217-22; the Luminex XMAP bead array system (www.luminexcorp.com); and the like, each of which are herein incorporated by reference.

The signature pattern may be generated from a biological sample using any convenient protocol, for example as described below. The readout may be a mean, average, median or the variance or other statistically or mathematically-derived value associated with the measurement. The cytokines readout information may be further refined by direct comparison with the corresponding reference or control pattern. A binding pattern may be evaluated on a number of points: to determine if there is a statistically significant change at any point in the data matrix; whether the change is an increase or decrease in the cytokines; and the like. The absolute values obtained for each cytokine under identical conditions will display a variability that is inherent in live biological systems.

Following obtainment of the signature pattern from the sample being assayed, the signature pattern is compared with a reference or control profile to make a prognosis regarding the phenotype of the patient from which the sample was obtained/derived. Typically a comparison is made with a sample or set of samples from an unaffected, normal source. Additionally, a reference or control signature pattern may be a signature pattern that is obtained from a sample of a patient known to have an autoimmune disease of interest, and therefore may be a positive reference or control profile.

In certain embodiments, the obtained signature pattern is compared to a single reference/control profile to obtain information regarding the phenotype of the patient being assayed. In yet other embodiments, the obtained signature pattern is compared to two or more different reference/control profiles to obtain more in depth information regarding the phenotype of the patient. For example, the obtained signature pattern may be compared to a positive and negative reference profile to obtain confirmed information regarding whether the patient has the phenotype of interest.

Samples can be obtained from the tissues or fluids of an individual. For example, samples can be obtained from whole blood, tissue biopsy, serum, etc. Other sources of samples are body fluids such as synovial fluid, lymph, cerebrospinal fluid, bronchial aspirates, and may further include saliva, milk, urine, and the like. Also included in the term are derivatives and fractions of such cells and fluids. Diagnostic samples are collected any time after an individual is suspected to have an autoimmune disease or has exhibited symptoms that predict such a disease.

Various immunoassays designed to quantitate cytokines may be used in screening. Measuring the concentration of the target protein in a sample or fraction thereof may be accomplished by a variety of specific assays. For example, a conventional sandwich type assay may be used in an array, ELISA, RIA, etc. format. A sandwich assay may first attach specific anti-cytokine antibodies to an insoluble surface or support. The particular manner of binding is not crucial so long as it is compatible with the reagents and overall methods of the invention. They may be bound to the plates covalently or non-covalently.

The insoluble supports may be any compositions to which polypeptides can be bound, which is readily separated from soluble material, and which is otherwise compatible with the overall method. The surface of such supports may be solid or porous and of any convenient shape. Examples of suitable insoluble supports to which the receptor is bound include slides, beads, *e.g.* magnetic beads, membranes and microtiter plates. These are typically made of glass, plastic (*e.g.* polystyrene), polysaccharides, nylon or nitrocellulose.

Patient sample preparations may then added to an antibody containing substrate. Preferably, a series of standards, containing known concentrations of the test protein is assayed in parallel with the samples or aliquots thereof to serve as controls. Preferably, samples are assayed in multiple spots, wells, etc. so that mean values can be obtained for each. The incubation time should be sufficient for binding, generally, from about 0.1 to 3 hr is sufficient. After incubation, the insoluble support is generally washed of non-bound components. A dilute non-ionic detergent medium at an appropriate pH, generally 7-8, can be used as a wash medium. From one to six washes can be employed, with sufficient volume to thoroughly wash non-specifically bound proteins present in the sample.

After washing, a solution containing a detection reagent, e.g. antibodies reactive with the cytokine, is applied. The second stage reagent may be labeled to facilitate direct or indirect quantification of binding. Examples of labels that permit direct measurement of second receptor binding include radiolabels, such as $^3$H or $^{125}$I, fluorescers, dyes, beads, chemiluminescers, colloidal particles, and the like. Examples of labels that permit indirect measurement of binding include enzymes where the substrate may provide for a colored or fluorescent product. In a preferred embodiment, the antibodies are labeled with a covalently bound enzyme capable of providing a detectable product signal after addition of suitable substrate. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art. The incubation time should be sufficient for the labeled ligand to bind available molecules. Generally, from about 0.1 to 3 hr is sufficient, usually 1 hr sufficing.

After the second binding step, the insoluble support is again washed free of non-specifically bound material, leaving the specific complex formed between the patient cytokines and the detection reagent. The signal produced by the bound conjugate is detected by conventional means. Where an enzyme conjugate is used, an appropriate enzyme substrate is provided so a detectable product is formed.

Other immunoassays are known in the art and may find use as diagnostics. Ouchterlony plates provide a simple determination of antibody binding. Western blots may be performed on protein gels or protein spots on filters, using a detection system specific for the autoimmune disease associated polypeptide as desired, conveniently using a labeling method as described for the sandwich assay.

In some cases, a competitive assay will be used. In addition to the patient sample, a competitor to the cytokine is added to the reaction mix. The competitor and the cytokine compete for binding. Usually, the competitor molecule will be labeled and detected as previously described, where the amount of competitor binding will be proportional to the amount of target antigen present. The concentration of competitor molecule will be from about 10 times the maximum anticipated protein concentration to about equal concentration in order to make the most sensitive and linear range of detection.

Alternatively, a reference sample may be used as a comparator. In such a case, the reference sample is labeled with or detected using a spectrally distinct fluorophore from that used to label or detect cytokines from the patient sample. This reference sample is mixed with the patient sample, and the mixed sample analyzed on arrays or another measurement methodology. Such an approach provides a ratio of patient: reference sample binding for an individual cytokine, thereby enabling direct comparative analysis of binding relative to reference sample binding.

For multiplex analysis of cytokines, arrays containing one or more anti-cytokine antibodies can be generated. Such an array is constructed comprising antibodies against cytokines, and may include antibodies binding cytokines listed in Table 2. Various immunoassays designed to quantitate cytokines may be used in screening. Measuring the concentration of the target protein in a sample or fraction thereof may be accomplished by a variety of specific assays. For example, a conventional sandwich type assay may be used in an array, ELISA, RIA, bead array, *etc.* format. A sandwich assay may first attach specific autoantigen peptides to an insoluble surface or support. The particular manner of binding is not crucial so long as it is compatible with the reagents and overall methods of the invention.

DIAGNOSTIC ALGORITHMS

An algorithm that combines the results of multiple antibody specificity and/or cytokine level determinations and that will discriminate robustly between individuals in different classifications with respect to autoimmune disease, *e.g.* rheumatoid arthritis, and controls for confounding variables and evaluating potential interactions is used for prognostic purposes.

The quantitation of markers in a test sample is determined by the methods described above and as known in the art. The quantitative data thus obtained is then subjected to an analytic classification process. In such a process, the raw data is manipulated according to an algorithm, where the algorithm has been pre-defined by a training set of data, for example as described in the examples provided herein. An algorithm may utilize the training set of data provided herein, or may utilize the guidelines provided herein to generate an algorithm with a different set of data.

An analytic classification process may use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms; etc.

Using any one of these methods, an autoimmune dataset is used to generate a predictive model. In the generation of such a model, a dataset comprising control and diseased samples is used as a training set. A training set will contain data for each of the markers of interest.

The predictive models demonstrated herein utilize the results of multiple protein level determinations, and provide an algorithm that will classify with a desired degree of accuracy an individual as belonging to a particular state, where a state may be autoimmune or non-autoimmune. Classification of interest include, without limitation, the assignment of a sample to one or more of the autoimmune disease states: i) autoimmune state vs. non-autoimmune state, (ii) pre-disease vs. normal, (iii) progression to severe vs. mild disease, (iv) active versus inactive disease, (v) need for repeat evaluations at a short or long interval, (vi) treatment versus no treatment, (vii) response to treatment with a specific DMARD versus a biologic agent versus a small molecule inhibitor such as MTX, (viii) need for additional tests versus no need for additional tests.

Classification can be made according to predictive modeling methods that set a threshold for determining the probability that a sample belongs to a given class. The probability preferably is at least 50%, or at least 60% or at least 70% or at least 80% or higher. Classifications also may be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model may be evaluated according to its ability to provide a quality metric, *e.g.* AUC or accuracy, of a particular value, or range of values. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold may refer to a predictive model that will classify a sample with an AUC (area under the curve) of at least about 0.7, at least about 0.75, at least about 0.8, at least about 0.85, at least about 0.9, or higher.

As is known in the art, the relative sensitivity and specificity of a predictive model can be "tuned" to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity may be at least about at least about 0.7, at least about 0.75, at least about 0.8, at least about 0.85, at least about 0.9, or higher.

The raw data may be initially analyzed by measuring the values for each marker, usually in triplicate or in multiple triplicates. The data may be manipulated, for example, raw data may be transformed using standard curves, and the average of triplicate measurements used to calculate the average and standard deviation for each patient. These values may be transformed before being used in the models, e.g. log-transformed, Box-Cox transformed (see Box and Cox (1964) J. Royal Stat. Soc., Series B, 26:211-246), etc. The data are then input into a predictive model, which will classify the sample according to the state. The resulting information may be transmitted to a patient or health professional.

In one embodiment, hierarchical clustering is performed in the derivation of a predictive model, where the Pearson correlation is employed as the clustering metric. One approach is to consider a patient atherosclerosis dataset as a "learning sample" in a problem of "supervised learning". CART is a standard in applications to medicine (Singer (1999) Recursive Partitioning in the Health Sciences, Springer), which may be modified by transforming any qualitative features to quantitative features; sorting them by attained significance levels, evaluated by sample reuse methods for Hotelling's $T^2$ statistic; and suitable application of the lasso method. Problems in prediction are turned into problems in regression without losing sight of prediction, indeed by making suitable use of the Gini criterion for classification in evaluating the quality of regressions.

This approach has led to what is termed FlexTree (Huang (2004) PNAS 101:10529-10534). FlexTree has performed very well in simulations and when applied to SNP and other forms of data. Software automating FlexTree has been developed. Alternatively LARTree or LART may be used. Fortunately, recent efforts have led to the development of such an approach, termed LARTree (or simply LART) Turnbull (2005) Classification Trees with Subset Analysis Selection by the Lasso, Stanford University. The name reflects binary trees, as in CART and FlexTree; the lasso, as has been noted; and the implementation of the lasso through what is termed LARS by Efron et al. (2004) Annals of Statistics 32:407-451. See, also, Huang et al. (2004) Tree-structured supervised learning and the genetics of hypertension. Proc Natl Acad Sci U S A. 101 (29):10529-34.

Other methods of analysis that may be used include logic regression. One method of logic regression Ruczinski (2003) Journal of Computational and Graphical Statistics 12:475-512. Logic regression resembles CART in that its classifier can be displayed as a binary tree. It is different in that each node has Boolean statements about features that are more general than the simple "and" statements produced by CART.

Another approach is that of nearest shrunken centroids (Tibshirani (2002) PNAS 99:6567-72). The technology is k-means-like, but has the advantage that by shrinking cluster centers, one automatically selects features (as in the lasso) so as to focus attention on small numbers of those that are informative. The approach is available as PAM software and is widely used. Two further sets of algorithms are random forests (Breiman (2001) Machine Learning 45:5-32 and MART (Hastie (2001) The Elements of Statistical Learning, Springer). These two methods are already "committee methods." Thus, they involve predictors that "vote" on outcome.

To provide significance ordering, the false discovery rate (FDR) may be determined. First, a set of null distributions of dissimilarity values is generated. In one embodiment, the values of observed profiles are permuted to create a sequence of distributions of correlation coefficients obtained out of chance, thereby creating an appropriate set of null distributions of correlation coefficients (see Tusher *et al.* (2001) PNAS **98**, 5116-21, herein incorporated by reference). The set of null distribution is obtained by: permuting the values of each profile for all available profiles; calculating the pair-wise correlation coefficients for all profile; calculating the probability density function of the correlation coefficients for this permutation; and repeating the procedure for N times, where N is a large number, usually 300. Using the N distributions, one calculates an appropriate measure (mean, median, *etc.*) of the count of correlation coefficient values that their values exceed the value (of similarity) that is obtained from the distribution of experimentally observed similarity values at given significance level.

The FDR is the ratio of the number of the expected falsely significant correlations (estimated from the correlations greater than this selected Pearson correlation in the set of randomized data) to the number of correlations greater than this selected Pearson correlation in the empirical data (significant correlations). This cut-off correlation value may be applied to the correlations between experimental profiles.

Using the aforementioned distribution, a level of confidence is chosen for significance. This is used to determine the lowest value of the correlation coefficient that exceeds the result that would have obtained by chance. Using this method, one obtains thresholds for positive correlation, negative correlation or both. Using this threshold(s), the user can filter the observed values of the pairwise correlation coefficients and eliminate those that do not exceed the threshold (s). Furthermore, an estimate of the false positive rate can be obtained for a given threshold. For each of the individual "random correlation" distributions, one can find how many observations fall outside the threshold range. This procedure provides a sequence of counts. The mean and the standard deviation of the sequence provide the average number of potential false positives and its standard deviation.

In the development of a predictive model, it may be desirable to select a subset of markers, *i.e.* at least 2, at least 3, at least 4, at least 5, up to the complete set of markers. Usually a subset of markers will be chosen that provides for the needs of the quantitative sample analysis, *e.g.* availability of reagents, convenience of quantitation, *etc.*, while maintaining a highly accurate predictive model.

The selection of a number of informative markers for building classification models requires the definition of a performance metric and a user-defined threshold for producing a model with useful predictive ability based on this metric. For example, the performance metric may be the AUC, the sensitivity and/or specificity of the prediction as well as the overall accuracy of the prediction model.

Also described herein are reagents and kits thereof for practicing one or more of the above-described methods. The subject reagents and kits thereof may vary greatly. Reagents of interest include reagents specifically designed for use in production of the above described expression profiles of circulating protein markers associated with autoimmune conditions.

One type of such reagent is an array or kit of antibodies that bind to a marker set of interest. A variety of different array formats are known in the art, with a wide variety of different probe structures, substrate compositions and attachment technologies. Representative array or kit compositions of interest include or consist of reagents for quantitation of at least two, at least three, at least four, at least five or more markers are selected from IP-10 (CXCL-10), MCP-1, CRP, eotaxin, GM-CSF, FGF-2, Flt-3 ligand, IL-10, IL-12 p40, IL-12 p70, IL-15, IL-1α, IL-1β, IL-6, and TNFα.

The kits may further include a software package for statistical analysis of one or more phenotypes, and may include a reference database for calculating the probability of classification. The kit may include reagents employed in the various methods, such as devices for withdrawing and handling blood samples, second stage antibodies, ELISA reagents; tubes, spin columns, and the like.

In addition to the above components, the subject kits will further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention which will be limited only by the appended claims.

As used herein the singular forms "a", "and", and "the" include plural referents unless the context clearly dictates otherwise. All technical and scientific terms used herein have the same meaning as commonly understood to one of ordinary skill in the art to which this invention belongs unless clearly indicated otherwise.

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (e.g. amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

EXPERIMENTAL

EXAMPLE 1

Development and optimization of methods for cytokine proofing in RA.

Cytokine profiling

Optimized methods were developed for profiling cytokines in human sera using a bead-array system (Luminex®). It has developed and validated methods to use HeteroBlock® (Omega Biologicals) to minimize non-specific bridging of capture and detection antibodies by rheumatoid factor and other heterophilic antibodies in bead-array systems (Figures 1). Using optimized methods, cytokine and chemokine analysis on 58 randomly selected serum samples derived from the ARAMIS inception cohort, as well as a cohort of RA, other inflammatory arthritis (ankylosing spondylitis and psoriatic arthritis), and healthy control samples from the Stanford Arthritis Center (Figures 2 and 3). Cytokine profiling demonstrated broad elevations of blood cytokines in approximately 25% of RA patients (Figures 2 and 3). Data were analyzed using Kruskal-Wallis tests with Dunn's adjustment for multiple comparisons, and elevated levels of TNF, IL-1 alpha, IL-12p40 and IL-13, and the chemokines eotaxin/CCL11, MCP-1/CCL2 and IP-10/CXCL10, were present in early RA as compared to control patients ($p < 0.0001$). Blood cytokines, including TNFα, IL-1, IL-6 and IL-12, were also elevated in a subset samples derived from RA patients as compared to controls (Figure 3).

Synovial microarrays and optimized bead-array cytokine profiling methodologies were further applied to identify peripheral blood autoantibody and cytokine profiles that characterize subgroups of patients with early RA (RA of < 6 month's duration). Autoantibody targeting of citrullinated epitopes was associated with elevated blood levels of TNFα, IL-1α, IL-6, IL-13, IL-15 and GM-CSF. Elevated proinflammatory cytokines and anti-citrulline autoantibodies were associated with clinical parameters predictive for progression to more severe arthritis, including elevations in CRP levels and possession of the shared epitope (SE) MHC polymorphism (Figure 3). Thus, proteomic analysis of serum autoantibodies, cytokines and chemokines enables stratification of early RA patients into molecular subgroups.

EXAMPLE 2

Studies were performed to determine what biomarkers are present in the pre-clinical period of disease in subjects that subsequently developed RA. These studies were accomplished using a U.S. military cohort in which 83 individuals who developed RA and had serial samples prior to the development of disease stored in the Department of Defense Serum Repository (DoDSR) were identified. De-identified demographic and relevant clinical data were abstracted from the chart, and serum stored in the DoDSR was recovered and sent to Denver for analysis. Demographic data on these 83 individuals with RA is presented in Table 3.

Table 3. Demographic and clinical characteristics of cases with rheumatoid arthritis **and serum samples available.**

| **Cases** (N = 83) | | |
| --- | --- | --- |
| Age at diagnosis, Mean (std) | 39.9 (10.0) | |
| Male, N (%) | 49 (59%) | |
| Race, N (%) | | |
| White | 57 (69%) | |
| Black | 21 (25%) | |
| Other | 5 (6%) | |
| Erosions, N (%) | | |
| Present | 42 (51 %) | |
| Absent | 34 (41 %) | |
| Unclassified | 7 (8%) | |
| RF positivity at or after diagnosis* | | |
| Sero-positive | 67 (81 %) | |
| Sero-negative | 13 (16%) | |
| Unclassed | 3 (4%) | |
| **Pre-Clinical Serum Samples** (N =243) | Mean (std) | Range |
| Number of samples per case | 2.9 (1.2) | (1 - 4) |
| Years before diagnosis of first sample | 6.6 (3.7) | (0.1-13.7) |
| *Based on case military chart review<br>std = standard deviation | | |

In the first series of experiments, 243 serial pre-diagnosis serum samples from these 83 subjects with RA were examined for the presence of RF as measured by nephelometry and anti-CCP antibody. 57% and 62% of subjects had at least one pre-diagnosis sample positive for RF or anti- CCP, respectively. Gender and race were not associated with the prevalence or timing of pre-clinical antibody appearance. Therefore, although this is a cohort that is enriched for males (59% of subjects), the findings do not appear to be gender-specifiic. Interestingly, one observation of potential importance to developing risk estimates for future development of disease was that, as age at the time of diagnosis of RA increased, the duration of pre-clinical antibody positivity increased. For example, as calculated by interval censored survival analysis, RF and anti-CCP were present for a median of 3.3 and 2.8 years, respectively, in subjects aged 20-29 at the time of diagnosis, and for a median of 8.6 and 6.4 years in subjects over the age of 50. In addition, in no subjects whose timing of autoantibodies and symptoms could be accurately determined did the time of symptom onset precede the appearance of RF or anti-CCP antibodies. Finally, antibodies demonstrated significant stability, with only 3/47 RF and 3/51 anti-CCP antibodies reverting to negative at any point during the pre-clinical period.

*Identification of antibody problems that predict development of RA*

Utilizing the same military RA cohort described above, in a case-control analysis it was found that the presence of 2 or more RA-related autoantibodies was associated with a high-risk for development of RA. If 2 or more antibodies were present, sensitivity for development of RA was 69% and specificity was 98%. This information is highly informative in building prediction models for RA development. Multiplex antibody profiling was performed using synovial antigen microarrays to further characterize autoantibody responses in pre-disease samples derived from patients that ultimately developed RA (Figure 7). Synovial microarrays contain 100+ proteins and peptides representing candidate autoantigens in RA, including: collagens type I, II, III, IV, VI, IX and XI, and overlapping peptides derived from type II collagen;

glycoprotein (GP)-39 and overlapping peptides derived from GP-39; the endoplasmic reticulum molecular chaperone BiP; native and deiminated fibrin, fibrinogen, and vimentin; native and citrulline-substituted filaggrin peptides including CCP1 and CCP2; native and citrulline-substituted fibrinogen and vimentin peptides; hnRNP-B1 and -D; heat shock proteins 65 and 70, and additional candidate autoantigens are being added in an ongoing basis. We applied synovial arrays to survey the specificity and diversity of the autoantibody response in the pre-disease samples in the U.S. military RA Cohort. Arrays are incubated with 1:150 dilutions of patient sera, washed, and then incubated with a fluorescently-labeled anti-human IgG or anti-human IgM secondary antibody to detect autoantibody binding. A laser microarray scan is then used to quantitate the fluorescence at each antigen feature on the microarrays, and then feature reactivities are statistically analyzed to identify individual or combinations of antibody specificities with value for predicting future development of RA.

Synovial micoarray analysis was performed on serial serum samples from asymptomatic subjects derived from the above described U.S. military pre-disease cohort, obtained at two time points (tp) preceding clinical RA and one tp post diagnosis (n =27), as well as from age and sex-matched controls (n = 27). Samples from RA cases were selected for CCP-negative status at tp1 (mean 5.2 yrs. pre-diagnosis), sero-converting to CCP-positive status at tp2 (mean 1.6 yrs. pre-diagnosis) and maintenance of CCP-positive status at tp3 (mean 2.9 yrs. post-diagnosis). All sera were analyzed using antigen microarrays that included 130 distinct peptides derived from > 20 RA candidate antigens, and a bead-based multiplex assay to measure serum cytokine concentrations, as described previously. Significance analysis of microarrays (SAM) was used to analyze differences in relative serum protein levels, and hierarchical clustering was performed to interrogate relationships of SAM-identified variables.

Two-class SAM comparisons of CCP-negative samples with matched controls at tp1 revealed a trend towards targeting of native epitopes derived from Vitronectin, Fibrinogen A, and H2B, and the citrullinated epitopes Fibrinogen A(616-635)cit, H2A(1-20)cit and ApoE(277-296)cit (Figure 8). At tp2, the now CCP-positive samples demonstrated additional targeting of citrullinated epitopes H2A(1-20)cit and Vimentin (58-77)cit as well as citrullinated Filaggrin peptides (false discovery rate (FDR) < 20%). At tp3, a similar signature as observed at tp2 was present, now including Vimentin (1-20)cit and H2A(1-20)cit (FDR < 14%) (Figure 8). Moreover, a panel of cytokines and chemokines associated and tightly clustered with cases at tp2 and 3, including several proinflammatory cytokines, IL-10, FGF-2 and Flt-3L. Relative elevations of 8 cytokines were observed at tp2, and of 12 cytokines at tp3. In CCP-negative pre-disease samples from subjects who progress to RA, several native and citrullinated epitopes were targeted. Over time, the immune response against citrullinated but not native epitopes broadened.

The data (Figures 4 and 8) suggest that a few native and citrullinated peptides are targeted in very early stages of preclinical disease, likely even before CCP2-ELISA sero-conversion. Later, anti-citrulline autoantibodies exhibit epitope spreading in pre-clinical and early RA, suggestive of affinity maturation (Figure 8). Further, a time course-dependent cytokine signature evolves that predominantly associates with increased targeting of citrullinated autoantigens and suggests an intimate co-regulation of cytokines and autoantibodies in pre-clinical RA Figures 4, 7 and 8).

Further integration and coupling of antibody analysis with cytokine/chemokine analysis and/or genetic analysis and/or other clinical and laboratory data will provide even greater sensitivity and predictive value for an individual patient progressing to clinically definite RA. Datasets from measurement of antibody, cytokine/chemokine, genetic and other laboratory values will be analyzed to determine the sensitivity and specificity of single and combinations of autoantibody, cytokine/chemokine and genetic markers for predicting subsequent development of RA and/or development of severe RA. Multiplex analysis of antibodies, cytokines/chemokines and other laboratory parameters is anticipated to provide a powerful tool for identifying individuals likely to develop RA and thus enabling physicians to institute early therapeutic intervention.

Example 3

Blood cytokine, chemokine, and c-reactive protein levels in the pre-clinical period of RA development

The following experiments were performed to determine whether blood cytokine, chemokine, or other inflammatory biomarkers such as c-reactive protein were present in asymptomatic individuals that are at risk for future development of RA.

Data are shown that focus on determining the relationship between cytokine/chemokine changes and the appearance of RA-related antibodies prior to the onset of clinically-apparent RA (Figures 4 and 5). For this initial analysis, 16 subjects with sero-positive RA were selected from the RA military cohort because they had at least two pre-diagnosis serum samples for analysis, with the earliest sample a median of 4.2 years prior to diagnosis. These earliest samples were all negative for anti-CCP, and nine were negative for all RF testing (including nephelometry and ELISA for IgM, IgA, IgG). Fourteen cytokines and chemokines were selected due to their known association with active RA and were measured using bead-based multiplex assay with the Heteroblock (TM) reagent also included to minimize the impact of RF. Multiple cytokine levels were elevated in RA cases when compared to matched military controls (Figure 4). Also, pre-clinical cytokine/chemokine levels from 13 subjects with sero-negative RA (RF and anti-CCP) were compared to matched controls and significant differences were not observed (Figure 5).

In this analysis, it was found that eotaxin, IL-1, GM-CSF, and MCP-1 were significantly elevated in the earliest pre-clinical samples from RA cases when compared to controls. Importantly, in the 9 cases whose initial sample was negative for both anti-CCP and RF antibodies, and thus free of any confounding by RF, IL-1 levels were elevated versus controls (p=0.04). In contrast, cytokine/chemokine levels in multiple pre- and post-diagnosis samples from patients with seronegative RA were found to not differ from controls.

*Cytokine/chemokine elevations prior to the diagnosis of RA.*

For this series of experiments, we performed multiplex cytokine/chemokine analysis on pre-diagnosis stored serum samples for a military cohort with known RA (Table 3).

For the initial experiment, to determine estimates of the timing of abnormal cytokine/chemokine and CRP levels, samples from military cases with RF or anti-CCP positivity in the pre-diagnosis period were divided into 1-year intervals covering the pre-diagnosis period, starting at the time of diagnosis and moving backwards in time. Median cytokine and chemokine levels from all cases with samples available during a given 1-year time period compared with their matching control samples from the same time period. For the interval 0-1 years prior to diagnosis, 27/57 (47%) cases had samples available. When compared to matched controls, the median levels of the following cytokines and chemokines were significantly elevated (P=<0.05) in the cases' serum samples during this 0-1 year interval (see Figure 6): IL-1a, IL-10, IL-6, IL-10, IL-12 p40, FGF-2, IP-10, and TNF$\alpha$. In addition, CRP was also significantly elevated in cases versus controls for the 0-1 pre-diagnosis interval.

For the interval 1-2 years prior to diagnosis, 26/57 (46%) cases had samples available for analysis. For this interval, cases had significant elevations in levels of the following cytokines and chemokines when compared to matched controls (Figure 6): IL-12 p40, FGF-2, and IP-10.

*Cytokine/chemokine elevations prior to the appearance of anti-CCP antibodies.* Of the RA cases with either RF or anti-CCP positive during the pre-clinical period, 28 had the following sequence of pre-diagnosis samples available - an initial sample that was anti-CCP negative, followed by an anti-CCP positive pre-diagnosis sample. The initial anti-CCP negative samples were collected a mean of 5.3 years prior to diagnosis and a mean of 2.5 years prior to the pre-diagnosis anti-CCP positive samples. Of these 28 cases, 14 (50%) were RF positive in their initial anti-CCP negative pre-diagnosis sample.

In the 28 initial anti-CCP negative samples, the following chemokines were significantly elevated in cases when compared to matched controls: IP-10 (CXCL-10) and MCP-1 (see Table 4). A subset analysis of the 14/28 cases that were negative for all antibodies in their initial pre-diagnosis sample revealed no significant differences in levels of cytokines and chemokines when compared to controls.

**Table 4. Cytokine and chemokine levels in pre-diagnosis samples prior to the appearance of anti-cyclic citrullinated peptide antibodies in RA patients.**

| Cytokines/Chemokines | Cases Median (25, 75% quartiles) | Controls Median (25, 75% quartile) | P-value |
|---|---|---|---|
| IP-10 (CXCL-10) | 114 (64,226) | 66 (31, 99) | 0.02 |
| MCP-1 | 144 (65, 208) | 67 (43, 150) | 0.01 |
| Trends towards differences were seen between cases and controls in the levels of the following inflammatory markers and cytokines/chemokines: CRP, eotaxin, GM-CSF, FGF-2, Flt-3 ligand, IL-10, IL-12 p40, IL-12 p70, IL-15, IL-1$\alpha$, IL-1$\beta$, IL-6, and TNF$\alpha$ | | | |

*Cytokine/chemokine elevations in pre-clinical samples from sero-negative RA cases.* 13 sero-negative RA cases were identified from the larger military RA cohort of 83 cases. Cytokine and chemokine levels were evaluated in these cases during both the pre- and post-diagnosis period. No significant differences in levels of cytokines and chemokines were seen in these sero-negative cases when compared to matched controls (Figure 5).

With these initial positive data, current analyses are focused on determining the relative timing of appearance of RA-related autoantibodies to these as well as other biomarkers, with the goal of defining a pattern that is highly predictive of future disease onset.

To this end, the next experiment was performed in order to determine the relationship between RA-related antibody elevation and specific elevations of cytokines/chemokines or c-reactive protein during the pre-clinical period of RA development with the goal of identifying a pattern that would be useful for prediction of impending disease in asymptomatic individuals with RA-related autoantibodies. Multiplex cytokine analysis (Figure 6) was performed on pre-diagnosis stored serum samples from RA military subjects (Table 3) who developed rheumatoid factor (RF) and/or anti-cyclic citrullinated peptide (anti-CCP) antibody during the pre-clinical period of RA development. Cytokine/chemokine levels were measured by bead-based assay in the presence of HeteroBlock™ to minimize effects of RF.

The determination of abnormal levels of cytokines/chemokines and CRP (>0.73 mg/dL) was made using receiver operator curve (ROC) analysis of post-diagnosis RA samples compared to matched controls, with a specificity for RA of >90%. This high specificity was chosen in order to maximize the predictive value of a given CRP or cytokine/chemokine value for true RA-related inflammation, although if lower cut-off levels for CRP or cytokines/chemokines are used, the relative timing of appearance of CRP, cytokines/chemokines, and antibodies can change. Using this high-specificity cut-off to determine positive/negative status of cytokines/chemokines and CRP, the timing of appearance of these biomarkers in relationship to antibody positivity was determined using interval censored survival analysis (SAS 9.1).

The prevalence of high specificity (>90%) pre-clinical cytokine/chemokine and CRP elevations in 66 military RA subjects with RF and/or anti-CCP positivity is reported in Table 5. As for timing of appearance of antibodies and cytokines/chemokines/CRP during the pre-clinical period: RF isotypes IgM, IgA, and IgG appeared a median of 3.8, 3.2, and 0.9 years prior to diagnosis, respectively. Anti-CCP appeared a median of 3.3 years prior to diagnosis. The median times of appearance of CRP and all cytokines/chemokines tested were within 2 years of diagnosis (Figure 8), although a subgroup including IP-10, IL-12p40, Flt-3 Ligand and IL-1alpha appeared earlier than the others, with a median appearance of >1.5 years . This data suggests that specific elevations in CRP and cytokines/chemokines can be used to predict impending clinically-apparent disease.

Table 5. Prevalence of Pre-Clinical Cytokine/Chemokine and C-Reactive Protein Elevations in 66 Military RA Subjects with Rheumatoid Factor and/or Anti-Cyclic Citrullinated Peptide Antibody Positivity

| Cytokine/Chemokine | N (% of 66) |
|---|---|
| C-reactive protein | 20 (30%) |
| IL-6 | 24 (36%) |
| IL-1alpha | 34 (52%) |
| IL-1beta | 30 (45%) |
| FgF-2 | 29 (44%) |
| IL-12p70 | 26 (39%) |
| IL-10 | 22 (33%) |
| Eotaxin | 9 (14%) |
| IL-15 | 31 (47%) |
| IP-10 | 39 (59%) |
| IL-12p40 | 38 (58%) |
| GM CSF | 28 (42%) |
| TNFalpha | 31 (47%) |
| Flt-3 Ligand | 38 (58%) |
| MCP-1 | 26 (40%) |

In the next experiment, the prevalence of rates of pre-clinical elevations of cyokines/chemokines/CRP was assessed in all 83 Military RA subjects, using cut-offs for each cytokine/chemokine and/or CRP that were >90% for RA. Of the 83 RA subjects, 76 had elevation/s of one or more cytokines/chemokines/CRP during the pre-clinical period, with the majority of elevations occurring in individuals positive for RF and/or anti-CCP. Prevalence rates are reported in Table 6, and suggest that elevations of multiple cytokine/chemokine/CRP during the pre-clinical period are common.

Table 6. Prevalence of Pre-Clinical Cytokine/Chemokine and/or C-Reactive Protein Elevations in 83 Military RA Subjects with or without Pre-Clinical Antibody Positivity

| Number of cytokines/chemokines/CRP positive during pre-clinical period | N % - out of 83 subjects Cut-off for positivity >90% specificity for RA) |
|---|---|
| 0 | 7 (8.4%) |
| 1 | 6 (7.2%) |
| 2 | 7 (8.4%) |
| 3 | 9 (10.8%) |
| 4 | 4 (4.8%) |
| 5 | 7 (8.4%) |
| 6 | 4 (4.8%) |
| 7 | 5 (6.0%) |
| 8 | 4 (4.8%) |
| 9 | 2 (2.4%) |
| 10 | 7 (8.4%) |
| 11 | 2 (2.4%) |
| 12 | 3 (3.6%) |
| 13 | 3 (3.6%) |
| 14 | 6 (7.2%) |
| 15 | 1 (1.2%) |
| 1-5 | 33 (43.4%) |
| 6-10 | 22 (28.9%) |
| 11-15 | 15 (20%) |

EXAMPLE 3

*Pre-clinical autoantibodies, cytokines/chemokines, and c-reactive protein* as *predictors of* RA *severity*

In this series of experiments, pre-clinical positivity for autoantibodies RF and anti-CCP, and elevations of cytokines/chemokines and CRP were evaluated for their ability to predict RA severity as measured by the presence of erosive disease.

In the first experiment, the RA military cohort was used to determine if RF or anti-CCP positivity predicted erosive RA. 51 subjects were positive for anti-CCP during the pre-clinical period of RA development. Pre-clinical anti-CCP positivity was strongly associated with the development of erosive RA (Adjusted odds ratio 11.3; 95% CI 2.3, 55.8; p=0.003). Pre-clinical RF positivity was not associated with increased risk for erosive disease. After controlling for anti-CCP positivity in regression analysis, no pre-clinical elevation of individual or combinations of cytokines/chemokines or CRP was associated with increased risk for erosive disease.

In an additional experiment, utilizing alternative cut-offs for cytokine/chemokine/CRP positivity that maximized both sensitivity and specificity for disease when compared to controls, pre-clinical anti-CCP positivity was significantly associated with increased pre-diagnosis levels of IL-12p40, IP-10, and FGF-2, suggesting that these markers may have a biologic relationship with anti-CCP positivity (Table 7).

Table 7. Cytokine/Chemokine Associations with Anti-Cyclic Citrullinated Peptide (anti-CCP) Positivity During the Pre-Clinical Period of Rheumatoid Arthritis Development

| Cytokine/Chemokine | Odds Ratio (95% CI; p-value) |
|---|---|
| IL-12p40 | 5.15 (1.62, 16.38; p<0.01) |
| IP-10 | 3.12 (1.01, 9.68; p=0.05) |

(continued)

| Cytokine/Chemokine | Odds Ratio (95% CI; p-value) |
|---|---|
| FGF-2 | 3.73 (1.26, 11.08; p=0.02) |

EXAMPLE 4

*Methods for establishing cut-off levels for cytokine%hemokine positivity during the pre-clinical period of RA-related autoimmunity.*

Utilizing the military RA cohort described above, analysis is currently underway to determine optimal cut-off values for each cytokine and chemokine for the prediction of autoimmunity. Using area under the curve (AUC) analysis and other techniques, the sensitivity and specificity of various levels of cyokines and chemokines will be analyzed and the optimal level for maximal diagnostic accuracy obtained. Using these cut-off levels for each cytokine and chemokine, currently healthy subjects at risk for the development of RA development can be identified. Data obtained from analysis of this military cohort and similar cohorts can then be applied to other cohorts such as the first-degree relative (FDR) cohort described above to determine risk for development of clinically-apparent RA.

The AUC or similar analyses can be used to determine the optimal cut-off values for individual cytokines and chemokines, combinations of cytokines/chemokines, and in expanded analysis, the optimal combinations of genetic markers, environmental exposures and/or questionnaire-obtained information, physical examination findings, autoantibody profiles, cytokine and chemokine elevations, and other biomarkers for the prediction of RA development. In addition, AUC or similar analyses can be used to identify which biomarkers and clinically-obtained data or combinations thereof will be most useful in the identification of subjects at sufficiently high risk of development of RA that preventive or pre-clinical treatment measures can be implemented.

EXAMPLE 5

*Autoantibodies, cytokines/chemokines, and c-reactive protein testing in the pre-clinical period of RA development are highly specific for the future development of RA and can identify timing of onset of* disease

In this series of experiments, it is shown that combinations of autoantibody, cytokine/chemokine, and c-reactive protein (CRP) positivity in the pre-clinical period of RA development is 1) highly specific for the future development of RA and 2) can predict what pre-diagnosis time interval an individual with autoantibody positivity is in. This approach is useful not only to identify an individual at high-risk for future development of RA, but also to determine at what time point future symptoms of RA may develop.

For these experiments, we utilized the military RA cohort described above. We selected from this cohort 73 cases that were determined to have sero-positive RA either by clinical RF testing in the military or by our own testing for RF and CCP in available post-diagnosis samples. The demographics of these 73 cases are reported in Table 8.

**Table 8. Demographic and clinical characteristics of sero-positive military rheumatoid arthritis cases and controls**

| | Sero-Positive RACases (N=73) | Controls (N=73) | Chi-squared testing |
|---|---|---|---|
| **Age at diagnosis, mean (std)[range]** | | | |
| | 39.9(10.3)[20.9-66.1] | 39.9 (10.3)[20.9-66.1] | P=1.00 |
| **Male** | 43 (58.9%) | 43 (58.9%) | P=1.00 |
| **Race** | | | |
| White | 49 (67.1 %) | 49(67.1%) | P=1.00 |
| Black | 19 (26.0%) | 19 (26.0%) | P=1.00 |
| Other* | 5 (6.9%) | 5 (6.9%) | P=1.00 |
| Erosions, **N** (%) | | | |
| Present | 37 (50.0%) | n/a | n/a |

(continued)

| Age at diagnosis, mean (std)[range] | Sero-Positive RACases (N=73) | Controls (N=73) | Chi-squared testing |
|---|---|---|---|
| Pre-Diagnosis Serum Samples (N =212 samples) | | | |
| Number of samples per case, Mean (std)[rangel | 2.2 (1.1)[1-4] | 2.2 (1.1)[1-4] | P=1.00 |
| Time collected before diagnosis (years), Mean (std)franqel | 4.7 (3.4)[5 days to 13.7 years] | 5.8 (4.0)[13 days to 13.7 years] | P=0.78 |

*Other includes Asian, Native American, and multi-racial subjects
ABBREVIATIONS: std = standard deviation; RA = rheumatoid arthritis

We then determined the prevalence rates of single and combination autoantibody positivity in all of the pre-diagnosis serum samples available for these 73 cases and their matched controls (Table 9). In addition, we determined the sensitivity, specificity, area-under-the-curve (AUC), and positive and negative predictive values (PPV, NPV, respectively) of single and combination autoantibody positivity for the classification of future RA (Table 9). PPV and NPV were calculated using a hypothetical population of 10,000 individuals with an estimated prevalence of RA of 1 %. This results show that combination autoantibody testing results in high specificity and high PPV for future onset of RA. In particular, the antibody profile of anti-CCP and/or 2 or more RF assays resulted in the highest sensitivity, specificity, and AUC.

**Table 9. Prevalence rates of single autoantibodies and combinations of autoantibodies determined in all pre-diagnosis rheumatoid arthritis (RA) case and control samples.**

| | Case Samples (N=212 samples in 73 cases) | Control Samples (N=212 samples in 73 controls) | P-value* | Sensitivity | Specificity | Area Under the Curve (AUC) | PPV NPV |
|---|---|---|---|---|---|---|---|
| **Autoantibodies** | | | | | | | |
| RFNeph (>24.4 Units/mL) | 68 (32.1 %) | 9 (4.2%) | <0.01 | 32.1 % | 95.8% | 0.64 | 7.1% 99.3% |
| RF-IgM (>13.6 IU/mL) | 80 (37.7%) | 5 (2.4%) | <0.01 | 37.7% | 97.6% | 0.68 | 13.7% 99.4% |
| RF-IgA (>10.5 IU/mL) | 73 (34.4%) | 5 (2.4%) | <0.01 | 34.4% | 97.6% | 0.66 | 12.3% 99.3% |
| RF-IgG (> 10.9 IU/mL) | 41 (19.3%) | 6 (2.8%) | <0.01 | 19.3% | 97.2% | 0.58 | 6.4% 99.2% |
| Anti-CCP (>5 U/mL) | 94 (44.3%) | 0 | <0.01 | 44.3% | 100% | 0.72 | 100% 99.4% |
| **Autoantibody Combinations** | | | | | | | |
| 2 or more RFs (any assay) | 68 (32.1%) | 2 (0.9%) | <0.01 | 32.1% | 99.1% | 0.66 | 26.4% 99.3% |

(continued)

| Autoantibody Combinations | | | | | | | |
|---|---|---|---|---|---|---|---|
| Anti-CCP and/or 2 or more RFs | 105 (49.5%) | 2 (0.9%) | <0.01 | 49.5% | 99.1% | 0.74 | 36.0% 99.5% |

*P-value represents the significance of difference in prevalence of antibodies between cases and controls with chi-squared testing.

**Sensitivity and specificity are of the antibody/combination positivity for case versus control status.

***Area under the curve values indicate ability of antibody test to distinguish between case and control status.

****Positive predictive value indicates probability that an individual with antibody (or profile) positivity will develop RA and was calculated using a hypothetical population of 10,000 individuals with a prevalence rate of RA of 1%. Negative predictive value indicates the probability that if an individual does not have antibody (or profile) positivity that they will develop RA, and was calculated using the same hypothetical population. ABBREVIATIONS: CRP = c-reactive protein; AUC = area under the curve; PPV = positive predictive value; NPV = negative predictive value; anti-CCP = anti-cyclic citrullinated peptide antibodies; RF = rheumatoid factor (any assay includes RF by nephelometry or RF isotypes IgM, IgA, IgG by ELISA).

We then determined the prevalence rates of single and combination cytokine/CRP positivity in all of the pre-diagnosis serum samples available for these 73 cases and their matched controls (Table 10). To determine cytokine/chemokine positivity, we performed AUC analysis as described in EXAMPLE 4 of the post-diagnosis sero-positive military cases and controls and determined a cut-off level for each cytokine/chemokine that was >90% specific for classification as an RA case. In addition, we determined the sensitivity, specificity, AUC, PPV, and NPV for combinations of cytokines/chemokines/CRP with and without autoantibodies (Table 11). This data shows that combination testing for autoantibodies, cytokines, and CRP in pre-diagnosis samples is highly specific for the future development of RA. However, as all pre-diagnosis samples were used for this analysis, these results cannot show how soon symptoms may develop in a subject with autoantibody, cytokine/chemokine, or CRP positivity.

Table 10. Prevalence rates of single cytokine and c‑reactive protein (CRP) positivity* and combinations determined in all pre-diagnosis rheumatoid arthritis (RA) case and control samples.

| Cytokines/CRP | Case Samples (N=212 samples in 73 cases) | Control Samples (N=212 samples in 73 controls) | P-value* |
|---|---|---|---|
| Eotaxin (≥610 pcg/mL) | 13 (6.1%) | 16 (7.5%) | 0.70 |
| FGF-2 (≥157 pcg/mL) | 4.4 (20.8%) | 19 (9.0%) | <0.01 |
| Flt-31 iqand(≥104 pcq/mL) | 69 (32.5%) | 50 (23.6%) | 0.51 |
| GM-CSF(≥141 pcg/mL) | 57 (26.9%) | 33 (15.6%). | <0.01 |
| IL-10(≥13 pcg/mL) | 39 (18.4%) | 21 (9.9%) . | 0.02 |
| IL-12-p40(≥85 pcg/mL) | 73 (34.4%) | 30(14.2%) | <0.01 |
| IL-12p70(≥12 pcg/mL) | 41 (19.3%) | 32 (15.1%) | 0.30 |
| IL-15(≥20 pcg/mL) | 48 (22.6%) | 19 (9.0%) | <0.01 |
| IL-1alpha(≥35 pcg/mL) | 70 (33.0%) | 42 (19.8%) | <0.01 |
| IL-1 beta(≥20 pcg/mL) | 45 (21.2%) | 20 (9.4%) | <0.01 |
| IL-6(≥55 pcg/mL) | 35 (16.5%) | 19 (9.0%) | 0.03 |
| IP-10(≥223 pcg/mL) | 59 (27.8%) | 38 (17.9%) | 0.02 |
| MCP-1(≥209 pcg/mL) | 41 (19.3%) | 34 (16.0%) | 0.45 |
| TNFalpha(≥135 pcg/mL) | 48 (22.6%) | 19 (9.0%) | <0.01 |
| CRP (>5 mg/L) | 42 (19.8%) | 20 (9.4%) | <0.01 |

(continued)

| Cytokine Combinations | | | |
| --- | --- | --- | --- |
| Zero cytokines | 39 (18.4%) | 85 (40.1 %) | <0.01 |
| >1 cytokine | 173 (81.6%) | 127 (59.9%) | <0.01 |
| >5 cytokines | 46 (21.7%) | 23 (10.8%) | <0.01 |
| >10 cytokines | 17 (8.0%) | 4 (1.9%) | <0.01 |

*A cut-off level for each of the cytokines was determined using receiver operator curve (ROC) analysis of post-RA diagnosis military case samples and matched controls. Cut-offs used were >90% specific for a diagnosis of RA. For CRP, a cut-off level of 5 mg/L was used.
**The p-value represents the significance of the difference of prevalence level of cytokine/CRP in cases versus controls (chi-squared testing).

**Table 11. Prevalence rates of combinations of autoantibody, cytokine, and c-reactive protein (CRP) positivity in all pre-diagnosis rheumatoid arthritis (RA) case and control samples.**

| Autoantibody, Cytokine/CRP combinations | Case Samples (N=212 samples In 73 cases) | Control Samples (N=212 samples in 73 controls) | P-value | Sensitivity | Specificity | AUC | PPV NPV |
| --- | --- | --- | --- | --- | --- | --- | --- |
| CRP positive | 42 (19.8%) | 20 (9.4%) | <0.01 | 19.8% | 90.6% | 0.55 | 2.1% 99.1% |
| Any cytokine positive (excluding CRP) | 173 (81.6%) | 127 (59.9%) | <0.01 | 81.6% | 40.1% | 0.61 | 1.4% 99.6% |
| ≥5 cytokines positive (excluding CRP) | 46 (21.7%) | 23 (10.8%) | <0.01 | 21.7% | 89.2% | 0.55 | 2.0% 99.1% |
| Anti-CCP and/or any 2 RFs and CRP | 27 (12.7%) | 1 (0.5%) | <0.01 | 12.7% | 99.5% | 0.56 | 21.0% 99.1% |
| Anti-CCP and/or any 2 RFs and any cytokine/ chemokine positive (excluding CRP) | 88 (41.5%) | 1 (0.5%) | <0.01 | 41.5% | 99.5% | 0.71 | 46.2% 99.4% |
| Anti-CCP and/or any 2 RFs and | 30 (14.2%) | 0 | <0.01 | 14.2% | 100% | 0.57 | 100% |

(continued)

| Autoantibody, Cytokine/CRP combinations | Case Samples (N=212 samples In 73 cases) | Control Samples (N=212 samples in 73 controls) | P-value | Sensitivity | Specificity | AUC | PPV NPV |
|---|---|---|---|---|---|---|---|
| Z5 cytokineslchemokines positive | | | | | | | 99.1% |

*P-value represents the significance of difference in prevalence of antibodies between cases and controls with chi-squared testing.

**Sensitivity and specificity are of the antibody/combination positivity for case versus control status.

***Area under the curve values indicate ability of antibody test to distinguish between case and control status.

****Positive predictive value indicates probability that an individual with antibody (or profile) positivity will develop RA and was calculated using a hypothetical population of 10,000 individuals with a prevalence rate of RA of 1%. Negative predictive value indicates the probability that if an individual does not have antibody (or profile) positivity that they will develop RA, and was calculated using the same hypothetical population. ABBREVIATIONS: CRP = c-reactive protein; AUC = area under the curve; PPV = positive predictive value; NPV = negative predictive value; anti-CCP = anti-cyclic citrullinated peptide antibodies; RF = rheumatoid factor (any assay includes RF by nephelometry or RF isotypes IgM, IgA. IgG by ELISA).

To address the issue of prediction of time to diagnosis of RA in the pre-clinical period, we divided the military case samples into four pre-diagnosis time intervals: 0-1 years pre-diagnosis, >1 to 5 years pre-diagnosis, >5 to 10 years pre-diagnosis, and >10 years prior to diagnosis. We then performed logistic regression analysis in the 212 samples from 73 sero-positive military RA cases to determine which of the pre-diagnosis time intervals the biomarkers were most strongly associated with. The results of this analysis are reported in Table 12. These results show that when analyzed separately both antibody positivity (anti-CCP and/or 2 or more RFs positive) and elevations of CRP and cytokines/chemokines are strongly associated with the immediate pre-diagnosis time interval 0-1 years rather than the more distant time intervals. In addition, the combination of antibody panel positivity with CRP or ≥5 cytokine/chemokine positivity led to the largest associations with the 0-1 years pre-RA diagnosis interval (odds ratios approximately 9 and 13, respectively for antibody panel and CRP or ≥5 cytokines positive).

**Table 12. Associations of combinations of autoantibodies and cytokines/CRP with pre-RA diagnosis time intervals.**

| | 0-1 Years Pre- RA Diagnosis | 1-5 Years Pre- RA Diagnosis | >5 Years Pre- RA Diagnosis | >10 years Pre-RA Diagnosis |
|---|---|---|---|---|
| Anti-CCP and/or 2 or more RFs | OR 8.33  95% CI 2.33-29.72  P<0.01 | OR 1.95  95% CI 0.70-5.44  P=0.20 | OR 0.96  95% CI 0.33-2.80  P=0.94 | Referent group |
| CRP positive (>5 mg/L) | 6.38  95% CI 1.23-33.04  P=0.03 | 1.54  95% CI 0.32-7.35  P=0.59 | 2.05  95% CI 0.42-9.99  P=0.38 | Referent group |
| ≥5 cytokines/ chemokines positive | 11.65  95% 1.35-100.16  P=0.03 | 4.91  95% CI 0.62-38.93  P=0.13 | 3.93  95% CI 0.48-32.34  P=0.20 | Referent group |
| Anti-CCP and/or 2 or | OR 8.69 | OR 2.38 | OR 2.22 | Referent group |

(continued)

|  | 0-1 Years Pre- RA Diagnosis | 1-5 Years Pre- RA Diagnosis | >5 Years Pre- RA Diagnosis | >10 years Pre-RA Diagnosis |
|---|---|---|---|---|
| more RFs *and* CRP positive | 95% CI 1.02-74.00 P=0.05 | 95% CI 0.29-19.32 P=0.42 | 95% CI 0.26-18.83 P=0.46 | |
| Anti-CCP and/or 2 or more RFs *and* ≥5 cytokines/ chemokines positive | OR 13.0 95% CI 1.55-109.00 P=0.02 | OR 3.80 95% CI 0.48-30.05 P=0.21 | OR 2.53 95% CI 0.30-21.22 P=0.39 | Referent group |
| ABBREVIATIONS: RA = rheumatoid arthritis; anti-CCP = anti-cyclic citrullinated peptide antibodies; RF = rheumatoid factor; CRP = c-reactive protein; OR = odds ratio; CI = confidence interval. | | | | |

In sum, this series of experiments demonstrates 1) the high specificity of pre-diagnosis antibody testing for future RA, 2) the additional increased specificity for future RA if cytokine/chemokine and CRP testing is added to antibody testing, and 3) the ability to use antibody, cytokine/chemokine, and CRP testing to classify individuals that are at high risk of future RA into pre-diagnosis time intervals. This approach can be used to classify individuals as being at 1) high risk for future RA and 2) within a specified time interval of potentially developing symptomatic disease. Such individuals with potentially incipient RA can then be evaluated for early therapeutic/preventive interventions.

EXAMPLE 6

Finally, a five-site study a cohort of 2100 first-degree relatives (FDRs) of probands with RA is recruited and evaluated serially for the presence of the same RA-related biomarkers. As this is the largest available type of population with the highest definable risk for future development of RA (3-5% based on estimated incidence rates of RA within FDRs) without additional screening techniques being employed, this cohort is considered appropriate to study for the presence of the same biomarkers and potentially predictive patterns.
These data allow a formal determination of the number of at-risk individuals who demonstrate an actionable profile of biomarkers. Discovery of biomarkers identifying individual who will progress from the asymptomatic state to clinical RA opens up the use of therapies that interrupt the development of this catastrophic disease at its earliest point by blocking the immune system prior to the onset of clinical symptoms.

**Claims**

1.  A method for the classification of an individual into subtypes, which subtypes are informative of the individual's probability of developing overt rheumatoid arthritis (RA), the method comprising:

    determining a cytokine signature pattern for increased levels of IL-6; and an autoantibody signature pattern for increased levels of at least 3 autoantibodies from a sample obtained from said autoimmune disease patient, wherein said autoantibodies bind to an antigen selected from citrullinated epitopes derived from histone 2A fibrinogen , fillagrin, vimentin, and ApoE
    comparing said cytokine and autoantibody signature pattern with a control signature pattern; wherein a statistically significant match with a positive pattern for a pre-disease subtype or a statistically significant difference from a normal pattern for said disease is indicative that said patient is at risk for developing overt rheumatoid arthritis.

2.  The method according to Claim 1, wherein said signature pattern comprises quantitative data for at least 2 cytokines selected from the group comprising or consisting of IP-10 (CXCL-10), MCP-1, CRP, eotaxin, GM-CSF, FGF-2, Flt-3 ligand, IL-10, IL-12 p40, IL-12p70, IL-15, IL-1α, IL-10, IL-6, and TNFα.

3.  The method according to Claim 1, wherein said signature pattern comprises quantitative data for at least 5 cytokines selected from the group comprising or consisting of IP-10 (CXCL-10), MCP-1, CRP, eotaxin, GM-CSF, FGF-2, Flt-3 ligand, IL-10, IL-12 p40, IL-12p70, IL-15, IL-1α, IL-10, IL-6, and TNFα.

**4.** The method of Claim 1, wherein said individual is classified in one of four pre-diagnosis time intervals: 0-1 years pre-diagnosis, >1 to 5 years pre-diagnosis, >5 to 10 years pre-diagnosis, and >10 years prior to diagnosis.

**Patentansprüche**

**1.** Verfahren zur Klassifizierung eines Individuums in Unterarten, wobei diese Unterarten Informationen über die Wahrscheinlichkeit geben, dass das Individuum offenen Gelenkrheumatismus (RA) entwickelt, wobei das Verfahren Folgendes umfasst:

das Bestimmen eines Zytokinsignaturmusters für erhöhte Mengen an IL-6 und eines Autoantikörpersignaturmusters für erhöhte Mengen an zumindest 3 Autoantikörpern aus einer Probe, die von dem Patienten mit der Autoimmunerkrankung stammt, worin die Autoantikörper an ein aus citrullinierten Epitopen, die von Histon 2A, Fibrinogen, Fillagrin, Vimentin und ApoE abgeleitet sind, ausgewähltes Antigen binden, das Vergleichen des Zytokin- und des Autoantikörpersignaturmusters mit einem Kontrollsignaturmuster, worin eine statistisch signifikante Übereinstimmung mit einem positiven Muster bezüglich einer Vorerkrankungsunterart oder eine statistisch signifikante Abweichung von einem normalen Muster für diese Erkrankung ein Indikator dafür ist, dass dieser Patient gefährdet ist, offenen Gelenkrheumatismus zu entwickeln.

**2.** Verfahren nach Anspruch 1, worin das Signaturmuster quantitative Daten zu zumindest 2 aus der aus IP-10 (CXCL-10), MCP-1, CRP, Eotaxin, GM-CSF, FGF-2, Flt-3-Ligand, IL-10, IL-12p40, IL-12p70, IL-15, IL-1$\alpha$, IL-1$\beta$, IL-6 und TNF$\alpha$ bestehenden Gruppe ausgewählten Zytokinen umfasst.

**3.** Verfahren nach Anspruch 1, worin das Signaturmuster quantitative Daten zu zumindest 5 aus der aus IP-10 (CXCL-10), MCP-1, CRP, Eotaxin, GM-CSF, FGF-2, Flt-3-Ligand, IL-10, IL-12p40, IL-12p70, IL-15, IL-1$\alpha$, IL-1$\beta$, IL-6 und TNF$\alpha$ bestehenden Gruppe ausgewählten Zytokinen umfasst.

**4.** Verfahren nach Anspruch 1, worin das Individuum einem von vier Zeitabständen vor der Diagnose zugeordnet wird: 0 bis 1 Jahr vor der Diagnose, > 1 bis 5 Jahre vor der Diagnose, > 5 bis 10 Jahre vor der Diagnose und > 10 Jahre vor der Diagnose.

**Revendications**

**1.** Procédé de classification d'un individu en sous-types, lesquels sous-types fournissent des informations sur la probabilité de l'individu de développer une polyarthrite rhumatoïde (PR) déclarée, le procédé consistant à :

déterminer un motif de signature de cytokine pour des taux augmentés d'IL-6 ; et un motif de signature d'auto-anticorps pour des taux augmentés d'au moins 3 auto-anticorps à partir d'un échantillon obtenu chez ledit patient présentant une maladie auto-immune, où lesdits auto-anticorps se lient à un antigène sélectionné parmi des épitopes citrullinés dérivés de l'histone 2A, du fibrinogène, de la filaggrine, de la vimentine, et de l'ApoE ; comparer ledit motif de signature de cytokine et d'auto-anticorps à un motif de signature de contrôle ; où une correspondance statistiquement significative avec un motif positif pour un sous-type pré-pathologique ou une différence statistiquement significative par rapport à un motif normal pour ladite pathologie indique que ledit patient est exposé à un risque de développer une polyarthrite rhumatoïde déclarée.

**2.** Procédé selon la revendication 1, dans lequel ledit motif de signature comprend des données quantitatives pour au moins 2 cytokines sélectionnées dans le groupe comprenant ou consistant en l'IP-10 (CXCL-10), MCP-1, la CRP, l'éotaxine, le GM-CSF, le FGF-2, le ligand de Flt-3, l'IL-10, l'IL-12 p40, l'IL-12 p70, l'IL-15, l'IL-1$\alpha$, l'IL-1$\beta$, l'IL-6 et le TNF$\alpha$.

**3.** Procédé selon la revendication 1, dans lequel ledit motif de signature comprend des données quantitatives pour au moins 5 cytokines sélectionnées dans le groupe comprenant ou consistant en l'IP-10 (CXCL-10), MCP-1, la CRP, l'éotaxine, le GM-CSF, le FGF-2, le ligand de Flt-3, l'IL-10, l'IL-12 p40, l'IL-12 p70, l'IL-15, l'IL-1$\alpha$, l'IL-15, l'IL-6 et le TNF$\alpha$.

**4.** Procédé selon la revendication 1, dans lequel ledit individu est classifié dans l'un de quatre intervalles de temps pré-diagnostic : 0-1 an pré-diagnostic, > 1 à 5 ans pré-diagnostic, > 5 à 10 ans pré-diagnostic, et > 10 ans avant le diagnostic.

FIG. 1

FIG. 2

FIG. 3A

| | Cytokine$^{high}$ cluster A (n=21) | Cytokine$^{high}$ cluster B (n=21) | p - value (Mann -Whitney U) |
|---|---|---|---|
| age | 54 | 53 | |
| CRP | 1.10 mg/dl | 0.33 mg/dl | p = 0.001 |
| ESR | 38.5 mm Hg | 19 mm Hg | p = 0.045 |
| RF titre | 285.0 | 32.1 | p = 0.0006 |
| CCP titre | 472.2 | 28.0 | p = 0.024 |
| HAQ | 1.25 | 1.125 | n.s. |
| GLOBAL | 33 | 45 | n.s. |

FIG. 3B

| Linear Correlation Analysis | | |
|---|---|---|
| Cytokine | Anti -CCP2 & cytokine R; (95% CI) | p |
| TNF alpha | 0.39 (0.15-0.59) | p = 0.003 |
| IL-10 | 0.38 (0.13-0.48) | p = 0.004 |
| IL-1 beta | 0.37 (0.12-0.58) | p = 0.005 |
| IL-1 alpha | 0.36 (0.10-0.57) | p = 0.007 |
| IL-2 | 0.36 (0.10-0.57) | p = 0.007 |
| IL-4 | 0.35 (0.10-0.56) | p = 0.008 |
| IL-12p70 | 0.34 (0.08-0.56) | p = 0.01 |
| IP-10/CXCL10 | 0.32 (0.06-0.54) | p = 0.02 |
| GM-CSF | 0.31 (0.05-0.53) | p = 0.02 |
| IFN gamma | 0.28 (0.014-0.50) | p = 0.04 |
| IL-6 | 0.22 (-0.05-0.46) | n.s. |
| IL-13 | 0.22 (-0.05-0.46) | n.s. |
| IL-15 | 0.22 (-0.05-0.46) | n.s. |
| Anti-CCP2 and RF titre: Spearman R = 0.76; CI0.62-0.86, p < 0.001 | | |

FIG. 3C

FIG. 4

FIG. 4 (Cont.)

FIG. 5

FIG. 5 (Cont.)

**Timing of Pre-Diagnosis CRP and Cytokine Elevations In Relationship to Antibody Appearance in Pre-Clinical Period of RA**

RF-IgM

CCP

RF-IgA

IP-10

IL-12p40

Flt-3 Ligand

IL-1alpha

IL-15

TNF-alpha

IL-1beta

RF-IgG

FGF-2

GM-CSF

IL-12p70

IL-6

MCP-1

IL-10

CRP

Eotaxin

0    0.5    1    1.5    2    2.5    3    3.5    4

**Years Prior to Diagnosis**

## FIG. 6

EP 2 158 326 B1

FIG. 7A

FIG. 7B

FIG. 8A

FIG. 8B

elim1 + 16-3
normalized to IgG
2calss: C-1 v. D-1
q < 27.2%
contrast 4.0

**FIG. 8C**

A052 H2B/e 1-20
A004 ApoE 277-296 cit
A030 FibrgnA616-635

low          high

elim1
normalized to IgG
2calss: C-2 v. D-2
q < 12.3%
contrast 6.0

A047 H2A/a 1-20 cit
A031 FibrgnA616-635cit
A075 hFibA616-635cit
A007 cf48-65-4cit
A117 Vim58-77cit
A015 Clusterin 386-405
A092 HSP65 261-271

**FIG. 8D**

low          high

elim1
normalized to IgG
2calss: C-3 v. D-3
q < 14.3%
contrast 6.0

A031 FibrgnA616-635cit
A075 hFibA616-635cit
A007 cf48-65-4cit
A110 Vim01-20cit
A117 Vim58-77cit
A047 H2A/a 1-20 cit
A032 Fibromodulin 103-122

**FIG. 8E**

low          high

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030003516 A1

### Non-patent literature cited in the description

- **Deane et al.** *Arthritis & Rheumatism,* September 2006, vol. 54 (9
- **Nielen et al.** *Ann Rheum Dis.,* April 2006, vol. 65 (4), 535-537
- **Kaplan SR ; Liang MH ; Luthra HS et al.** *The American Rheumatism Association,* 1987
- *Arthritis Rheum,* 1988, vol. 31, 315-324
- **Nielen MM ; van Schaardenburg D ; Reesink HW ; van de Stadt RJ ; van der Horst-Bruinsma IE ; de Koning MH et al.** Specific autoantibodies precede the symptoms of rheumatoid arthritis: a study of serial measurements in blood donors. *Arthritis Rheum.,* February 2004, vol. 50 (2), 380-6
- **Rantapaa-Dahlqvist S ; de Jong BA ; Berglin E ; Hallmans G ; Wadell G ; Stenlund H et al.** Antibodies against cyclic citrullinated peptide and IgA rheumatoid factor predict the development of rheumatoid arthritis. *Arthritis Rheum.,* October 2003, vol. 48 (10), 2741-9
- **Berglin E ; Padyukov L ; Sundin U ; Hallmans G ; Stenlund H ; Van Venrooij WJ ; Klareskog L ; Dahlqvist SR.** A combination of autoantibodies to cyclic citrullinated peptide (CCP) and HLA-DRB1 locus antigens is strongly associated with future onset of rheumatoid arthritis. *Arthritis Res Ther.,* 11 May 2004, vol. 6 (4), R303-8
- *Clinical and Experimental Rheumatology,* 2005, vol. 23 (39), S93-99
- **Doan, O.V. et al.** *J Manag Care Pharm.,* 2006, vol. 12 (7), 555-69
- *Clin Exp Rheumatol.,* November 2006, vol. 24 (6), S077-82
- *Clin Exp Rheumatol.,* 2006, vol. 24 (6), S077-82

- **van Dongen H ; van Aken J ; Lard JR et al.** Efficacy of methotrexate treatment in patients with probable rheumatoid arthritis: a double-blind, randomized, placebo-controlled trial. *Arthritis Rheum,* 2007, vol. 56 (5), 1424-1432
- **Green M ; Marzo-Ortega H ; McGonagle D et al.** Persistence of mild, early inflammatory arthritis: the importance of disease duration, rheumatoid factor, and the shared epitope. *Arthritis Rheum,* 1999, vol. 42 (10), 2184-8
- *N Engl J Med.,* 1993, vol. 329 (24), 1764-9
- *Neurol Sci.,* December 2003, vol. 24 (5), 298-300
- **Colyer et al.** *J Chromatogr A.,* 1997, vol. 781 (1-2), 271-6
- **Petricoin et al.** *Lancet,* 2002, vol. 359, 572-77
- **Chan-Hui.** *Clinical Immunology,* 2004, vol. 111, 162-174
- **Montagne.** *Eur J Clin Chem Clin Biochem.,* 1992, vol. 30 (4), 217-22
- **Box ; Cox.** *J. Royal Stat. Soc., Series B,* 1964, vol. 26, 211-246
- **Huang.** *PNAS,* 2004, vol. 101, 10529-10534
- **Efron et al.** *Annals of Statistics,* 2004, vol. 32, 407-451
- **Huang et al.** Tree-structured supervised learning and the genetics of hypertension. *Proc Natl Acad Sci U S A.,* 2004, vol. 101 (29), 10529-34
- **Ruczinski.** *Journal of Computational and Graphical Statistics,* 2003, vol. 12, 475-512
- **Tibshirani.** *PNAS,* 2002, vol. 99, 6567-72
- **Breiman.** *Machine Learning,* 2001, vol. 45, 5-32
- **Hastie.** The Elements of Statistical Learning. Springer, 2001